# EUROPÄISCHE PATENTSCHRIFT

(11) **EP 2 187 832 B1**
(45) Veröffentlichungstag und Bekanntmachung des Hinweises auf die Patenterteilung: **30.06.2021**
(21) Anmeldenummer: 07802797.6
(22) Anmeldetag: 22.08.2007
(51) Int. Cl.: A61B 50/20

(54) **TRÄGER ZUR AUFNAHME VON CHIRURGISCHEM MATERIAL**
CARRIER FOR SURGICAL MATERIAL AND BONE PLATES
SUPPORT POUR MATÉRIEL CHIRURGICAL ET PLAQUES D'OSTHÉOSYNTHÈSE

(43) Veröffentlichungstag der Anmeldung: 26.05.2010
(73) Patentinhaber: Medartis Holding AG, 4057 Basel (CH)
(72) Erfinder: KUHLMANN, Dirk, CH-6006 Luzern (CH); MICHEL, Thomas, CH-8006 Zürich (CH); EBERT, Marcus, 73527 Schwäbisch Gmünd (DE); ZUBERBÜHLER, Corina, 8004 Zürich (CH)
(74) Vertreter: Hepp Wenger Ryffel AG
(86) Internationale Anmeldenummer: PCT/EP2007/058725
(87) Internationale Veröffentlichungsnummer: WO 2009/024189

(56) Entgegenhaltungen:
- EP-A- 1 582 169
- WO-A-03/079918
- WO-A-2005/053753
- BE-A6- 1 005 149
- DE-U1- 20 108 390
- DE-U1-202005 010 530
- DE-U1-202007 004 638
- US-A- 5 215 726
- US-A- 6 164 442
- US-A1- 2007 034 630
- US-B1- 6 450 328
- US-B1- 7 066 341
- None

## Beschreibung

Die vorliegende Erfindung betrifft einen Träger zur Aufnahme von insbesondere chirurgischem Material, ein Aufnahmemodul für derartige Träger, eine Halterung zum Fixieren wenigstens eines Trägers für eine Knochenplatte, einen Träger für eine Knochenplatte, eine Einheit aus einem Träger und einer Knochenplatte, ein System aus einer Halterung zum Fixieren und einer derartigen Einheit sowie einen chirurgischen Behälter gemäss den Oberbegriffen der unabhängigen Patentansprüche.

In der Knochenchirurgie wird oftmals eine Vielzahl von Knochenschrauben, Implantaten und Instrumenten benötigt, welche dem Chirurgen bei der Operation nach Bedarf zur Verfügung stehen müssen. Die oftmals geringen Dimensionen der Knochenschrauben und Implantate erschweren dabei deren Handhabung sehr. Insbesondere ist es schwierig, kleine Knochenschrauben und Implantate mit der Hand oder einem Schraubenzieher zu ergreifen und ihr Herunterfallen zu verhindern. Weiterhin ist es bei den bekannten Systemen möglich, dass eine versehentlich entnommene Knochenschraube unbenutzt wieder in einen Behälter zurückgesetzt wird. Besonders im Hinblick auf die strengen Anforderungen an die Sicherheit und Sterilität ist dies ein nicht tolerierbarer Nachteil.

Aus WO 2005/092231 ist ein Träger für die Lagerung und Darbietung von einer Knochenschraube bekannt. Zur Entnahme der Knochenschraube muss zunächst ein Griff nach oben entfernt werden. Diese Anordnung weist jedoch vielerlei Nachteile auf. So ist etwa die Schraube bei aufgesetztem Griff nicht sichtbar, so dass beispielsweise nicht unmittelbar erkennbar ist, ob es sich um eine Torx- oder eine Kreuzschlitzschraube handelt oder welche Länge oder Durchmesser die Schraube aufweist. Ebenso ist eine allfällige Beschriftung mit Längenangaben auf der Oberseite des Trägers bei aufgesetztem Griff nicht sichtbar. Weiterhin erschwert der Griff die Sterilisation des Schraubenkopfes und führt zu einer vergrösserten Bauhöhe. Darüber hinaus muss der Griff bei der Entnahme der Knochenschraube separat abgelegt werden, was einen weiteren Arbeitsschritt darstellt und daher das Hantieren während einer Operation erschwert. Schliesslich ist es möglich, eine versehentlich entnommene Knochenschraube wieder zurück in den Träger einzusetzen und beispielsweise versehentlich in einer späteren Operation zu verwenden, was die Anforderungen an die Sterilität nicht erfüllt. Wenn stattdessen der Griff nach dem Einsetzen entnommen wird, ist die Schraube nicht mehr gesichert und kann herausfallen.

Aus WO 01/49198 ist ein elastischer Träger bekannt, in welchen eine Knochenschraube einklemmbar ist. Bei dieser Vorrichtung muss der Träger nach der Entnahme aus der Hand gelegt werden, was das Hantieren verzögert. Weiterhin ist auch in diesem Träger eine versehentlich entnommene Knochenschraube wieder einsetzbar. Die in WO 2004/096073 dargestellte Sterilverpackung für eine Knochenschraube weist einen Versiegelungsfilm auf, welcher vor der Entnahme der Knochenschraube etwa mit einem Schraubenzieher durchstossen werden muss. Hierzu muss die Verpackung in die Hand genommen werden, was auch hier ein zweihändiges Hantieren erfordert. Bei dieser Lösung kann es vorkommen, dass Reste des Versiegelungsfilms durch das Durchstossen am Implantat haften bleiben und diese versehentlich implantiert werden. Aus DE202007004638U ist ein Träger zur Aufnahme von mindestens einer Knochenschraube und/oder von mindestens einem Knochennagel gemäß dem Oberbegriff des Anspruchs 1 bekannt.

Es ist die Aufgabe der vorliegenden Erfindung, die Nachteile des Bekannten zu überwinden und insbesondere einen Träger bereitzustellen, der kostengünstig herstellbar ist, aus welchem etwa eine Knochenschraube leicht entnehmbar ist und welcher gewährleistet, dass eine versehentlich entnommene Knochenschraube, die beispielsweise beschädigt oder unsteril geworden ist, nicht wieder in den Träger eingesetzt werden kann. Darüber hinaus soll ein Träger geschaffen werden, welcher die eindeutige Kennzeichnung und daher die Verfolgbarkeit des chirurgischen Materials erlaubt.

Die Erfindung betrifft einen Träger zur Aufnahme von mindestens einer Knochenschraube und/oder von mindestens einem Knochennagel gemäß Anspruch 1. Weitere Aspekte der Erfindung werden in abhängigen Ansprüchen definiert. Ein erster Aspekt betrifft einen Träger zur Aufnahme von insbesondere chirurgischem Material. Bei dem Material kann es sich etwa um eine Knochenschraube, einen Knochennagel, einen Knochendübel, eine Klammer, einen Gewindestift, einen Stift oder einen Draht handeln. Der Träger weist eine Halterung auf, welche wahlweise in eine Halteposition oder in eine Freigabeposition bringbar ist. In der Halteposition ist das Material in der Halterung gehalten. Bei der Ausübung einer Kraft und/oder eines Drehmoments auf das Material und/oder auf die Halterung in einer ersten Richtung ist die Halterung von der Halteposition in die Freigabeposition bringbar. In dieser Freigabeposition ist das Material in einer zweiten Richtung entnehmbar, welche von der ersten Richtung verschieden ist.

Insbesondere kann es sich bei der Kraft um eine lineare Kraft handeln, welche in Richtung der Spitze einer Knochenschraube wirkt. Die zweite Richtung kann der ersten Richtung im Wesentlichen entgegengesetzt sein, also in Richtung des Kopfes der Knochenschraube. Auf diese Weise lässt sich die Knochenschraube besonders einfach durch Drücken und anschliessendes Ziehen aus dem Träger entnehmen. Alternativ kann der Träger derart ausgebildet sein, dass eine Zugkraft in Richtung des Kopfes der Knochenschraube erforderlich ist und die Knochenschraube anschliessend in Richtung ihrer Spitze entnehmbar ist. Ausserdem werden keine losen Bauteile freigelegt, welche in einem separaten Arbeitsschritt abgelegt werden müssen.

Gemäss einem Beispiel weist der Träger mindestens eine Haltefläche auf, die in der Halteposition in Kontakt mit dem Material ist. Bevorzugt sind die Halteflächen am Halteelement angeordnet. Durch diesen Kontakt, welcher einen Kraftschluss, Reibschluss und/oder einen Formschluss bewirkt, ist das Material in der Halterung gehalten. Dies verhindert beispielsweise das Herausfallen oder Verkanten einer Knochenschraube in dem Träger, wenn der sie enthaltende Behälter gekippt wird.

Alternativ kann mindestens eine Haltefläche insbesondere erst durch eine Bewegung des Materials in Kontakt mit dem Material bringbar sein. Das Material kann in diesem Fall locker im Träger eingesetzt sein und eine geringe Bewegung zulassen. Eine weitere Bewegung wird durch einen somit entstehenden Kontakt zwischen der Haltefläche und dem Material verhindert, was das vollständige insbesondere unbeabsichtigte Entfernen des Materials aus dem Träger verhindert. Beispielsweise kann sich die Haltefläche im Zwischenraum zwischen zwei oder mehr Gewindegängen einer im Träger eingesetzten Knochenschraube befinden, ohne diese oder den Schraubenkern zu berühren. Durch dieses Beipiel kann das Material geschont werden, da die Haltefläche nicht an das Material gedrückt wird.

Bevorzugt ist die Halterung bei der Ausübung einer Druckkraft auf das Material in der ersten Richtung von der Halteposition in die Freigabeposition bringbar. Dies stellt einen besonders einfachen und intuitiv bedienbaren Aufbau dar.

Alternativ hierzu ist es etwa möglich, dass bei Ausübung eines Drehmoments oder einer Kombination aus einer Druckkraft und einem Drehmoment das Material von der Halteposition in die Freigabeposition bringbar ist. Im Sinne der Erfindung kann unter einer Richtung auch eine Drehrichtung verstanden werden. Weiterhin ist im Sinne der Erfindung die zweite Richtung auch beispielsweise dann von der ersten Richtung verschieden, wenn ein Drehmoment um eine Achse - etwa um die Achse einer Knochenschraube - ausgeübt wird und die Knochenschraube entlang dieser Achse entnommen wird. Weiterhin ist es alternativ denkbar und liegt im Rahmen der Erfindung, dass das Material nach der Anwendung einer Drucckraft seitlich, also etwa in einer Richtung quer zu einem Schaft einer Knochenschraube, entnehmbar ist. Auch in diesem Falle ist die zweite Richtung von der ersten Richtung verschieden.

Bevorzugt weist der Träger ein Basiselement auf, welches relativ zur Halterung bewegbar ist. Dabei kann die Halterung insbesondere in der ersten Richtung relativ zum Basiselement bewegbar sein.

Vorteilhaft weist der Träger Federmittel auf, mittels welcher die Halterung zumindest in der Halteposition bezogen auf das Basiselement festklemmbar ist.

Besonders bevorzugt ist die Halterung bei der Ausübung einer Kraft auf das Material in der ersten Richtung derart relativ zum Basiselement bewegbar, dass die Halterung von der Halteposition in die Freigabeposition bringbar ist. Die Halteposition und die Freigabeposition werden somit durch die relative Position der Halterung und des Basiselements zueinander bestimmt.

Bevorzugt weist dabei die Halterung eine Kontaktfläche und das Basiselement eine Gegenkontaktfläche auf. Zumindest in der Halteposition sind dabei die Kontaktfläche und die Gegenkontaktfläche in Kontakt. Dieser Kontakt kann einen Kraftschluss, Reibschluss und/oder einen Formschluss darstellen. Weiterhin sind die Kontaktfläche und die Gegenkontaktfläche derart aufeinander abgestimmt, dass die Halterung bei der Ausübung einer Kraft auf das Material in der ersten Richtung in die Freigabeposition bewegbar ist. In der Freigabeposition können die Kontaktfläche und die Gegenkontaktfläche in Kontakt sein; alternativ dazu können die Kontaktfläche und die Gegenkontaktfläche in der Freigabeposition nicht in Kontakt sein. Mittels der Federmittel wird bei Erreichen der Freigabeposition die Halterung in eine Position gebracht, in der die Halteflächen das Material nicht mehr halten. Somit ist das Material in dieser Freigabeposition aus der Halterung entnehmbar, da kein Kraftschluss, Reibschluss oder Formschluss mehr besteht.

Vorteilhaft ist die Kontaktfläche als Oberfläche mindestens eines Nockens und/oder einer Abschrägung ausgebildet. Dies ermöglicht einen besonders einfachen Aufbau.

Alternativ hierzu kann die Freigabeposition durch die Zerstörung eines Bauteils erreicht werden. Beispielsweise kann der Träger Sollbruchmittel enthalten, welche bei der Ausübung einer Kraft und/oder eines Drehmoments zerstört werden und somit die Freigabe des Materials erlauben. Bevorzugt sind die Sollbruchmittel als geschwächte Bereiche ausgebildet. Gemäss einem Beispiel sind die Sollbruchmittel am Basiselement angeordnet.

Bevorzugt weist der Träger mindestens eine Führungsfläche auf, die mit mindestens einer Gleitfläche eines chirurgischen Behälters in Kontakt bringbar ist. Dies ermöglicht das Einsetzen eines oder mehrerer erfindungsgemässer Träger in einen Behälter. Bevorzugt ist die Gleitfläche als Oberfläche mindestens einer Führungslamelle ausgebildet, wobei die Führungsfläche und die Gleitfläche derart ausgebildet und aufeinander abgestimmt sind, dass der Träger entlang der Führungslamelle bewegbar ist. Vorteilhaft ist der Träger dabei in einen Zwischenraum zwischen zwei benachbarten Führungslamellen einsetzbar. Bevorzugt ist mindestens eine Führungsfläche am Basiselement angeordnet.

Die bewegbare Anordnung der Träger entlang mindestens einer Führungslamelle erlaubt die Realisierung eines so genannten FIFO-Mechanismus ("first in, first out"). Demgemäss können beispielsweise die an der Führungslamelle hinzuzufügenden Träger nur an einem ersten Ende der der Führungslamellen eingesetzt werden. Damit wird gewährleistet, dass immer der jeweils zuerst in die Gleitschiene eingefügte Träger entnommen wird. Die Anordnung der Träger in einer Gleitschiene erleichtert weiterhin das Zählen des verbleibenden Materials und ein allfälliges Nachbestellen, wenn sämtliche Träger an das eine der Enden der Gleitschiene bewegt werden.

Bevorzugt ist der Träger bezüglich mindestens einer der Führungslamellen in eine Einrastposition bringbar. Der Träger kann derart beschaffen sein, dass er beim Einsetzen in den Zwischenraum zwischen zwei Führungslamellen automatisch in diese Einrastposition gerät. In dieser Einrastposition ist der Träger nicht mehr aus dem Zwischenraum entfernbar.

Vorteilhaft weist der Träger Freigabemittel auf, welche derart ausgebildet und angeordnet sind, dass der Träger bei der Bewegung der Halterung von der Halteposition in die Freigabeposition bezüglich der Führungslamelle in eine Entnahmeposition gebracht werden kann. In dieser Entnahmeposition befindet sich der Träger nicht mehr in der Einrastposition und kann somit beispielsweise aus dem Zwischenraum zwischen zwei Führungslamellen entfernt werden. Durch diese Ausgestaltung kann der Träger nicht aus dem Zwischenraum entfernt werden, solange er noch das chirurgische Material enthält und sich in Folge dessen in der Halteposition befindet. Erst die Auslösung des Freigabemechanismus durch die Anwendung einer Kraft und damit die Überführung in die Freigabeposition führen dazu, dass der Träger aus dem Zwischenraum entnehmbar ist. Auf diese Weise werden also durch die Anwendung der Kraft gleichzeitig und automatisch zwei Mechanismen in Gang gesetzt. Bevorzugt steht die Haltefläche in direkter Wirkverbindung mit den Freigabemitteln. Insbesondere kann die Haltefläche an den Freigabemitteln angeordnet sein. Somit werden beide Freigabemechanismen durch ein einzelnes Bauteil vermittelt. Besonders bevorzugt sind die Freigabemittel als einstückiger Federdraht ausgebildet, dessen eines Ende die Haltefläche und das andere Ende die Führungsfläche zur lösbaren Verbindung mit einer Gleitfläche bildet.

Weiterhin bevorzugt weist der Träger Kennzeichnungen auf. Dabei kann es sich beispielsweise um Herstellerangaben und/oder eine Produktnummer und/oder technische Spezifikationen handeln. Zumindest Teile dieser Kennzeichnungen können vorteilhaft so angeordnet sein, dass sie sichtbar sind, wenn der Träger in einen chirurgischen Behälter, insbesondere in eine Gleitschiene in einem chirurgischen Behälter, eingesetzt ist. Alternativ oder zusätzlich ist es möglich, dass der Träger eine Seriennummer aufweist. Die Kennzeichnungen können etwa als direkt lesbare Zeichen wie beispielsweise Buchstaben und Zahlen oder als maschinenlesbarer Code ausgebildet sein. Auf diese Weise kann der Träger mit dem eingesetzten Material von der Herstellung bis zu seiner Verwendung verfolgt werden.

Bevorzugt weist der Träger Sperrmittel auf, welche wahlweise in eine Aufnahmeposition oder in eine Sperrposition bringbar sind. Dabei ist das Material in der Aufnahmeposition der Sperrmittel in den Träger einsetzbar oder eingesetzt. Die Sperrmittel sind derart ausgebildet, dass sie sich bei der Entnahme des Materials in die Sperrposition bewegen und dort verbleiben. In der Sperrposition ist das Material nicht mehr in den Träger einsetzbar. Somit kann wirkungsvoll verhindert werden, dass eine versehentlich entnommene und möglicherweise beschädigte und/oder verschmutzte Knochenschraube wieder in den Träger eingesetzt wird. Einerseits verhindert dieser Mechanismus die spätere Wiederverwendung einer möglicherweise unsteril gewordenen Knochenschraube. Andererseits wird der Gefahr entgegengetreten, dass die Knochenschraube in einen anderen als den ursprünglichen Träger eingesetzt wird, welcher eine andere Produktnummer oder technische Spezifikation aufweist.

Bevorzugt weist der Träger eine Aufnahmeöffnung zur Aufnahme zumindest eines Teils des Materials auf. Bei dem Teil des Materials kann es sich etwa um einen Teil eines Schaftes einer Knochenschraube handeln.

Bevorzugt sind die Sperrmittel als wenigstens ein Federelement ausgebildet. In der Aufnahmeposition dringt das Federelement nur so weit in einen Sperrbereich in der Aufnahmeöffnung ein, dass der Teil des Materials in den Sperrbereich der Aufnahmeöffnung eindringen kann. Bei der Entnahme des Materials dringt das Federelement derart weit in den Sperrbereich der Aufnahmeöffnung ein, dass der Teil des Materials nicht in den Sperrbereich der Aufnahmeöffnung eindringen kann. Durch das Eindringen des Federelements in den Sperrbereich bei der Entnahme des Materials wird somit die Sperrposition erreicht.

Weiterhin bevorzugt weist der Träger Verbindungsmittel auf, mittels welcher gleichartige, benachbarte Träger miteinander lösbar verbindbar oder verbunden sind. Dies ermöglicht es, mehrere Träger zu einer Einheit zu verbinden, was beispielsweise die Handhabung und das Nachfüllen eines chirurgischen Behälters erleichtert. Eine derartige Einheit kann etwa als Ganzes etwa in den Zwischenraum zwischen zwei Führungslamellen eingesetzt werden. Alternativ ist es möglich, durch Lösen der Verbindungsmittel nur einige der Träger gleichzeitig etwa in den Zwischenraum zwischen zwei Führungslamellen einzusetzen. Die Verbindungsmittel können etwa als Rastnase an einer ersten Seite des Trägers und komplementäre Aussparung an einer zweiten, der ersten Seite gegenüberliegenden Seite ausgebildet sein. Weiterhin ist es denkbar und liegt im Rahmen der Erfindung, dass die Verbindungsmittel als Sollbruchelemente ausgebildet sind, welche mehrere Träger miteinander verbinden.

Darüber hinaus betrifft die Erfindung einen Träger mit einer beliebigen Kombination der oben beschriebenen Merkmale mit darin eingesetztem Material, beispielsweise mit einer darin eingesetzten Knochenschraube, einem Knochennagel, einer Klammer, einem Gewindestift, einem Stift oder einem Draht.

Ein weiteres Beispiel betrifft ein Aufnahmemodul mit einer Aufnahme für mindestens einen Träger für insbesondere chirurgisches Material. Beispielsweise kann das Aufnahmemodul für die Aufnahme eines erfindungsgemässen Trägers geeignet sein. Das erfindungsgemässe Aufnahmemodul enthält Drehverbindungsmittel, welche derart ausgebildet und am Aufnahmemodul angeordnet sind, dass das Aufnahmemodul mit einem chirurgischen Behälter drehbar und lösbar verbindbar ist. Bevorzugt ist das Aufnahmemodul wahlweise in eine Lagerungsposition oder eine Bereitstellungsposition schwenkbar. Diese Ausgestaltung ermöglicht es, längliches Material wie etwa Knochenschrauben in einem chirurgischen Behälter zu lagern, deren Länge grösser ist als die Höhe des Behälters.

So kann beispielsweise eine Knochenschraube in der Lagerungsposition liegend angeordnet sein, während sie in der Bereitstellungsposition aufrecht angeordnet ist. Insgesamt kann somit eine geringere Bauhöhe eines chirurgischen Behälters erzielt werden.

Bevorzugt sind die Drehverbindungsmittel als Eingrifflöcher ausgebildet, welche mit Eingriffsvorsprüngen eines chirurgischen Behälters zusammenwirken können. Bevorzugt enthalten die Eingrifflöcher einen kreissegmentförmigen Abschnitt sowie einen Langlochabschnitt. Dieser Aufbau erlaubt eine Bereitstellungsposition, aus welcher das Aufnahmemodul nicht allein durch eine Drehbewegung, sondern nur mit einer zusätzlichen Hebebewegung bewegbar ist. Auf diese Weise ist das Aufnahmemodul unanfälliger gegenüber einer unbeabsichtigten Bewegung von der Bereitstellungsposition in die Lagerungsposition.

Alternativ können die Drehverbindungsmittel als Stifte ausgebildet sein. Die Stifte können beispielsweise in komplementäre Aussparungen in einer oder mehreren Seitenwänden des Behälters eingreifen.

Vorteilhaft weist das Aufnahmemodul mindestens eine Führungslamelle mit mindestens einer Gleitfläche auf. In den Zwischenraum zwischen zwei benachbarten Führungslamellen kann ein Träger eingesetzt werden, indem zumindest eine Führungsfläche des Trägers mit der Gleitfläche in Kontakt gebracht werden.

Bevorzugt sind die Drehverbindungsmittel derart ausgebildet und am Aufnahmemodul angeordnet, dass das mit dem Behälter drehbar verbundene Aufnahmemodul um eine Drehachse relativ zum Behälter drehbar ist, welche im Wesentlichen parallel zu einer Bodenplatte des Behälters ist.

Weiterhin bevorzugt ist die Aufnahme in einer Aufnahmerichtung ausgerichtet, die im Wesentlichen senkrecht zu einer Drehachse liegt, um welche das Aufnahmemodul drehbar mit dem Behälter verbindbar oder verbunden ist. Die Aufnahmerichtung wird dabei etwa durch die Hauptausdehnungsrichtung eines erfindungsgemäss im Aufnahmemodul eingesetzten Materials definiert. Die Hauptausdehnungsrichtung kann dabei beispielsweise die axiale Richtung einer Knochenschraube sein.

Ein weiteres Beispiel betrifft die Lagerung und Bereitstellung von Knochenplatten für die Knochenchirurgie. Herkömmliche hierfür vorgesehene Behälter - wie beispielsweise der in US 5,732,821 dargestellte - verfügen über Vertiefungen, welche den Formen der Knochenplatten angepasst sind und in denen eine oder mehrere Knochenplatten abgelegt werden können. Nachteilig ist hierbei einerseits, dass der Chirurg an die durch die Vertiefungen vorgegebene Auswahl an Knochenplatten gebunden ist. Der Behälter erlaubt somit keine individuelle Bestückung. Weiterhin ist das Ergreifen einer Knochenplatte erschwert, wenn diese tief in der Vertiefung versunken ist. Darüber hinaus kann es erforderlich sein, eine versehentlich entnommene Knochenplatte wieder in den Behälter zurückzulegen. Dies ist besonders nachteilig, da somit bereits berührte Knochenplatten wieder in den Behälter gelangen. Überdies ist eine effektive Sterilisation vor allem der sich im unteren Bereich der Vertiefung befindenden Knochenplatten schwierig. Schliesslich ist nicht unmittelbar erkennbar, wie viele Knochenplatten sich noch in einer Vertiefung befinden, was das Nachbestellen erheblich erschwert.

Daher ist es eine weitere Aufgabe der vorliegenden Erfindung, eine Vorrichtung zur Lagerung und Bereitstellung von Knochenplatten bereitzustellen und dabei die Nachteile des Bekannten zu vermeiden. Insbesondere sollte ein Behälter also individuell mit Knochenplatten bestückbar sein, und die Knochenplatten sollten leicht ergreifbar sein.

Diese Aufgabe wird zunächst durch eine Halterung zum Fixieren wenigstens eines Knochenplattenträgers für eine Knochenplatte auf einer Bodenplatte gelöst. Die Halterung weist einen Steg auf, der der im Wesentlichen senkrecht zu einer Grundebene der Halterung ausgerichtet ist. Bei der Grundebene handelt es sich um eine gedachte Ebene, welche etwa mit der Ebene einer Bodenplatte übereinstimmen kann, auf welcher die Halterung erfindungsgemäss fixiert ist. Auf einen derartigen Steg können mehrere der weiter unten beschriebenen, erfindungsgemässen Knochenplattenträger gestapelt werden.

Bevorzugt weist der Steg Kennzeichnungen auf, die in eindeutigem Zusammenhang zu ihrem jeweiligen Abstand von der Grundebene stehen. Bei den Kennzeichnungen kann es sich insbesondere um eine Skala handeln, welche eine aufsteigende und vorteilhaft bei eins beginnende Abfolge natürlicher Zahlen aufweist. Eine derartige Skala ermöglicht es, beim Zusammenwirken mit mindestens einem der weiter unten beschriebenen erfindungsgemässen Knochenplattenträger für eine Knochenplatte die Anzahl der auf der Halterung gestapelten Knochenplattenträger direkt abzulesen. Der Querschnitt des Steges kann beispielsweise flach, rund oder eckig sein.

Bevorzugt weist die Halterung Befestigungsmittel auf, mittels deren der Steg auf einer insbesondere gelochten Bodenplatte befestigbar ist.

Weiterhin bevorzugt verfügt die Halterung über ein flächiges Formelement, welches in der Grundebene oder parallel zu dieser liegt und welches im Wesentlichen die Form einer Knochenplatte wiedergibt. Bevorzugt ist das flächige Formelement derart ausgebildet und angeordnet, dass es beim erfindungsgemässen Fixieren auf einer Bodenplatte in Kontakt mit der Bodenplatte gerät. Die Wiedergabe der Form der Knochenplatte ermöglicht das unmittelbare Erkennen der Form der für diese Halterung vorgesehenen Knochenplatte. Darüber ist es möglich, eine Bodenplatte eines chirurgischen Behälters nach Bedarf mit einer Auswahl an verschiedenen, erfindungsgemässen Halterungen zu bestücken.

Ein weiteres Beispiel betrifft einen Knochenplattenträger für mindestens eine Knochenplatte, welche über mindestens eine obere Kontaktfläche und mindestens eine untere Kontaktfläche verfügt. Die obere und die untere Kontaktfläche sind derart ausgebildet, dass mehrere der Knochenplattenträger mit jeweils mindestens einer Knochenplatte übereinander stapelbar sind. Ein derartiger Aufbau erlaubt eine besonderes Platz sparende Anordnung mehrere Knochenplatten etwa in einem chirurgischen Behälter.

Bevorzugt enthält der Knochenplattenträger eine Grundfläche aus Kunststoff. Besonders bevorzugt kann es sich bei dem Kunststoff um hochtemperaturbeständige thermoplastische Kunststoffe wie Polyetheretherketon (PEEK) oder Polyphenylsulfon (PPSU) handeln. Bevorzugt wird die Grundfläche durch Ausschneiden hergestellt. Besonders bevorzugt kann dies durch Wasserstrahlschneiden, Laserschneiden oder mechanisches Schneiden mit Messern erfolgen. Vorteilhaft weist der Knochenplattenträger mindestens ein Halteelement auf, welches auf der Grundfläche angeordnet ist und an welchem eine Knochenplatte fixierbar ist. Die Halteelemente bestehen bevorzugt aus hochtemperaturbeständigen thermoplastischen Kunststoffe wie Polyetheretherketon (PEEK) oder Polyphenylsulfon (PPSU) und werden bevorzugt auf die Grundfläche aufgeschweisst, besonders bevorzugt durch Ultraschallschweissen, Kleben oder Stecken. Die Halteelemente werden bevorzugt im Spritzgussverfahren hergestellt.

Bevorzugt ist mindestens eines der Halteelemente zumindest teilweise elastisch ausgebildet. Dies ermöglicht ein einfaches Fixieren der Knochenplatte am Knochenplattenträger, insbesondere ein Festklemmen an den Halteelementen.

Bevorzugt weist das mindestens eine Halteelement einen Stützbereich und/oder mindestens ein insbesondere seitliches Schnappelement auf. Der Stützbereich kann insbesondere in Form eines zentralen Kegelstumpfes oder Pyramidenstumpfes ausgebildet sein. Der mindestens eine Stützbereich ist dabei derart ausgebildet und angeordnet, dass er mit einer Öffnung der Knochenplatte, insbesondere einer Aufnahmeöffnung für eine Knochenschraube, in Kontakt bringbar ist. Weiterhin ist das oder sind die Schnappelemente derart ausgebildet und angeordnet, dass damit eine auf dem Stützbereich angeordnete Knochenplatte gegen den Stützbereich drückbar ist.

Mit besonderem Vorteil weist der Knochenplattenträger mindestens eine Öffnung auf, welche derart ausgebildet und angeordnet ist, dass mehrere der Knochenplattenträger mit jeweils mindestens einer Knochenplatte derart übereinander stapelbar sind, dass die Öffnungen jeweils benachbarter Knochenplattenträger zueinander fluchtend ausgerichtet sind. Ein derartiger Aufbau ermöglicht das Stapeln mehrerer Knochenplattenträger auf dem Steg einer erfindungsgemässen Halterung.

Bevorzugt umfasst der Knochenplattenträger ein Griffelement zum Ergreifen des Knochenplattenträgers. Vorteilhaft weist das Griffelement im Wesentlichen die Form eines Kegelstumpfes auf. Insbesondere kann der Kegelstumpf hohl ausgebildet sein. Damit können die hohlen Kegelstümpfe gestapelter, benachbarter Knochenplattenträger ineinander eingreifen.

Bevorzugt kann die Öffnung in der Deckelfläche des Kegelstumpfes angeordnet und derart ausgebildet sein, dass bei übereinander gestapelten, gleichartigen Knochenplattenträgern die Öffnungen zueinander fluchtend ausgerichtet sind. Diese Konstruktion erlaubt eine besonders einfache Bauform.

Besonders bevorzugt weist der Knochenplattenträger Kennzeichnungen auf. Dabei kann es sich beispielsweise um Herstellerangaben und/oder eine Produktnummer und/oder technische Spezifikationen handeln. Alternativ oder zusätzlich ist es möglich, dass der Knochenplattenträger eine Seriennummer aufweist. Auf diese Weise kann der Knochenplattenträger von der Herstellung bis zu seiner Verwendung verfolgt werden. Die Kennzeichnungen können etwa als direkt lesbare Zeichen wie beispielsweise Buchstaben und Zahlen oder als maschinenlesbarer Code ausgebildet sein

Darüber hinaus betrifft die Erfindung eine Einheit aus einem erfindungsgemässen Knochenplattenträger für mindestens eine Knochenplatte und aus mindestens einer darauf aufgesetzten einen Knochenplatte.

Mit Vorteil ist mindestens eines der Halteelemente des Knochenplattenträgers der Einheit derart ausgebildet, dass die Knochenplatte mit mindestens einem der Halteelemente nur so weit in Kontakt ist, dass kein direkter Kontakt zwischen der Knochenplatte und der Grundfläche besteht. Dies verringert die Grösse der Kontaktfläche der Knochenplatte und vergrössert somit die freie Oberfläche der Knochenplatte, welche einer Sterilisation ungehindert zugänglich ist.

Besonders bevorzugt liegt die Knochenplatte zumindest teilweise auf einem Stützbereich des Halteelements auf. Alternativ oder zusätzlich ist die Knochenplatte seitlich von den seitlichen Schnappelementen eingeklemmt. Dies resultiert in einer besonders grossen freien Oberfläche der Knochenplatte, welche einer Sterilisation zur Verfügung steht.

Weiterhin betrifft die Erfindung ein System aus mindestens einer erfindungsgemässen Halterung zum Fixieren und mindestens einer Einheit aus einem erfindungsgemässen Knochenplattenträger und mindestens einer Knochenplatte.

Bevorzugt sind die Halterung zum Fixieren und der Knochenplattenträger derart ausgebildet und aufeinander abgestimmt, dass übereinander gestapelte Knochenplattenträger gemeinsam auf den Steg der Halterung aufsetzbar sind. Auf diese Weise kann der Steg in die zueinander fluchtend ausgerichteten Öffnungen der Knochenplattenträger eindringen und/oder sie durchdringen. Ein derartiges System ermöglicht die sortierte Bereitstellung mehrerer gleichartiger Knochenplatten.

Im Folgenden wird die Erfindung an Hand von Ausführungsbeispielen und mit Hilfe von Figuren erläutert. Es zeigen:

| | |
|---|---|
| Figuren 1a bis 1f | einen erfindungsgemässen Träger mit einer Knochenschraube in einer Halteposition; |
| Figuren 2a bis 2e | den erfindungsgemässen Träger aus den Figuren 1a bis 1e mit einer Knochenschraube in einer Freigabeposition; |
| Figur 3 | die Einzelteile des erfindungsgemässen Trägers; |
| Figur 4 | eine Explosionsdarstellung des erfindungsgemässen Trägers mit einer Knochenschraube; |
| Figuren 5a bis 5c | perspektivische Darstellungen zur Veranschaulichung der Bewegung von der Halteposition in die Freigabeposition; |
| Figuren 6a und 6b | Schnittzeichnungen zur Veranschaulichung der Bewegung von der Halteposition in die Freigabeposition; |
| Figuren 7a und 7b | Darstellungen einer Einheit aus vier miteinander verbundenen erfindungsgemässen Trägern; |
| Figuren 8a und 8b | eine schematische Darstellung einer alternativen Ausführungsform eines erfindungsgemässen Trägers; |
| Figuren 9a bis 9i | eine weitere Ausführungsform eines erfindungsgemässen Trägers; |
| Figuren 10a und 10b | in einen Zwischenraum zwischen zwei Führungslamellen eingesetzte erfindungsgemässe Träger; |
| Figur 11a und 11b | einen chirurgischen Behälter mit mehreren Führungslamellen und mit in deren Zwischenräumen eingesetzten erfindungsgemässen Trägern; |
| Figur 12a bis 12c | eine erfindungsgemässe Halterung zum Fixieren eines Knochenplattenträgers für eine Knochenplatte; |
| Figuren 13a bis 13d | einen erfindungsgemässen Knochenplattenträger mit einer darauf fixierten Knochenplatte; |
| Figuren 14a und 14b | zwei übereinander gestapelte erfindungsgemässe Knochenplattenträger mit jeweils einer darauf fixierten Knochenplatte; |
| Figuren 15a bis 15d | ein erfindungsgemässes System aus einer Halterung mit einer bzw. mehreren Einheiten aus jeweils einem Knochenplattenträger und einer Knochenplatte; |
| Figur 16a und 16b | einen chirurgischen Behälter mit mehreren auf einer Bodenplatte fixierten erfindungsgemässen Systemen aus jeweils einer Halterung mit mehreren Einheiten aus jeweils einem Knochenplattenträger und einer Knochenplatte; |
| Figuren 17a und 17b | einen chirurgischen Behälter mit zwei erfindungsgemässen Aufnahmemodulen; |
| Figuren 18a bis 18c | einen chirurgischen Behälter mit einem erfindungsgemässen Aufnahmemodul in einer Lagerungsposition; |
| Figuren 19a bis 19c | den chirurgischen Behälter aus Figur 18a mit dem erfindungsgemässen Aufnahmemodul in einer Bereitstellungsposition; |
| Figuren 20a und 20b | eine alternative Ausführungsform eines erfindungsgemässen Trägers mit Sperrmitteln. |

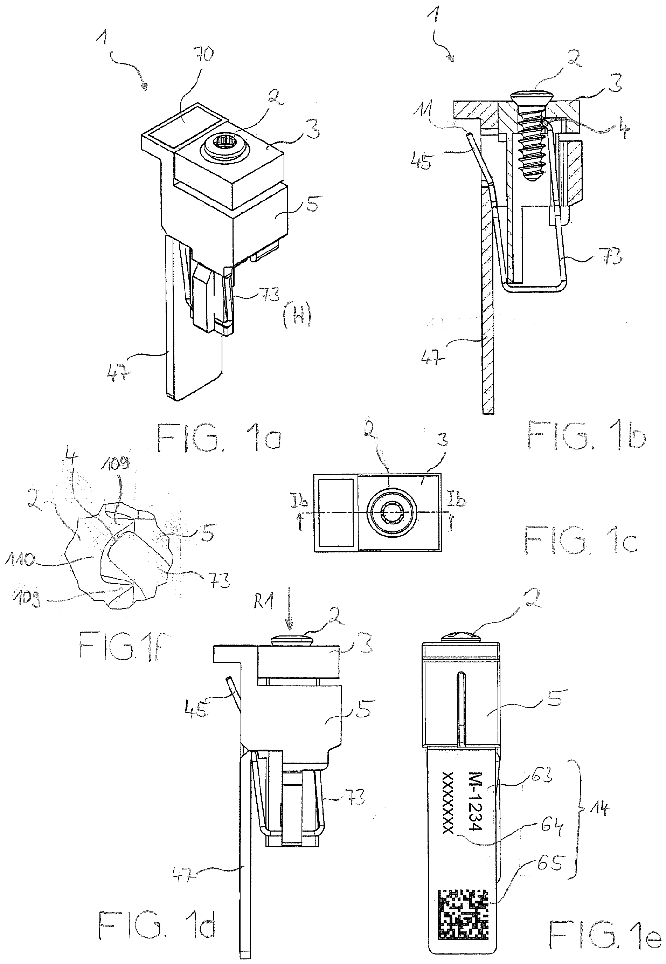

Figuren 1a bis 1e zeigen einen erfindungsgemässen Träger 1 in einer Halteposition H. Der Träger 1 umfasst ein Basiselement 5 und eine Halterung 3. In die Halterung 3 ist eine Knochenschraube 2 eingesetzt, welche ein chirurgisches Material darstellt. Der Träger 1 enthält eine Kennzeichenlasche 47, welche Kennzeichnungen 14 aufweist. Diese Kennzeichnungen 14 enthalten eine Produktbezeichnung 63, welche hier M-1234 lautet, eine Seriennummer 64, welche als xxxxxxx angedeutet ist, sowie einen 2D-Matrixcode 65. Diese Kennzeichnungen 14 können etwa als Laserbeschriftungen ausgebildet sein. Weiterhin weist der Träger 1 eine obere Kennzeichnungsfläche 70 auf, welche beispielsweise hier nicht dargestellte Produktinformationen wie etwa die Länge der Knochenschraube 2 wiedergeben kann.

Die Halterung 3 weist Freigabemittel auf, welche als Federdraht 73 ausgebildet sind. Am einen Ende des Federdrahts 73 ist eine Haltefläche 4 angeordnet, welche in der dargestellten Halteposition H in Kontakt mit der Knochenschraube 2 ist. Am anderen Ende des Federdrahts 73 ist eine Rastzunge 45 mit einer Führungsfläche 11 angeordnet, deren Funktion weiter unten erläutert wird. Auf diese Weise ist die Knochenschraube 2 in der Halterung 3 sowohl durch einen Kraftschluss als auch durch einen Reibschluss und Formschluss haltbar.

Bevorzugt, aber nicht zwingend, ist der Federdraht 73 so angeordnet, dass die Haltefläche 4 erst durch eine Bewegung der Knochenschraube 2 in Kontakt mit der Knochenschraube 2 gelangt. Gemäss der vergrösserten Darstellung in Figur 1f befindet sich die Haltefläche 4 im Freiraum zwischen zwei Gewindegängen 109 der Knochenschraube 2, ohne dass sie die Gewindegänge 109 oder den Schraubenkern 110 berührt. Die Knochenschraube 2 kann somit in axialer Richtung geringfügig bewegt werden. Auf diese Weise kann die Knochenschraube 2 nicht aus dem Träger 1 fallen, da sie noch an einer Flanke eines Gewindeganges 109 gehalten wird. Die Distanz zwischen Haltefläche 4 und Knochenschraube 2 kann dabei weniger als 0,05 mm betragen. Da die Knochenschraube 2 nicht seitlich eingeklemmt ist, kann sie nicht durch den Federdraht 73 beschädigt werden.

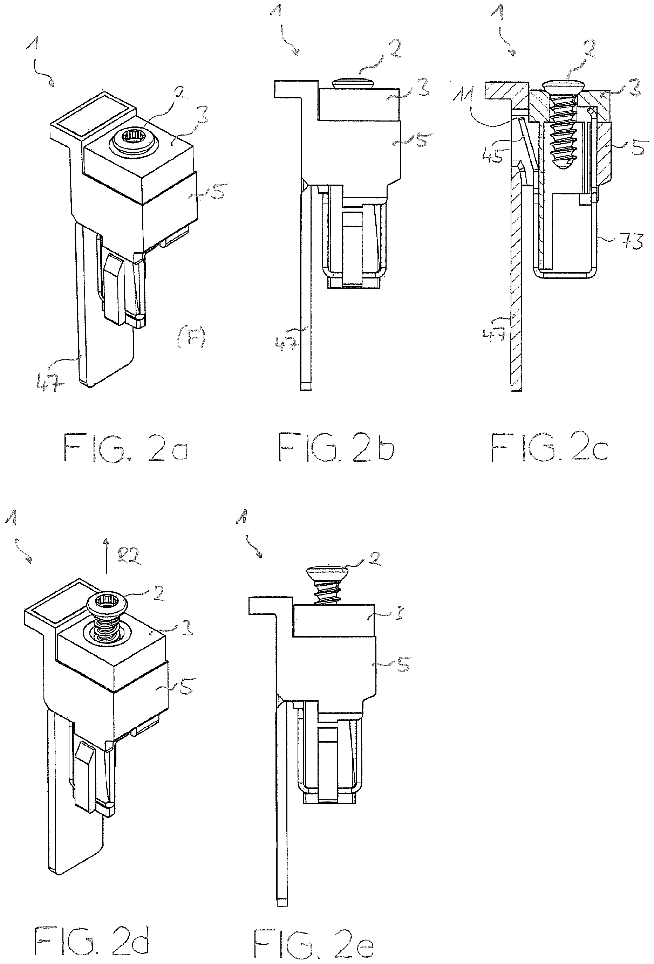

Durch Ausübung einer Kraft auf die Knochenschraube 2 und/oder die Halterung 3 in einer ersten Richtung R1 ist die Halterung 3 von der Halteposition H in die in den Figuren 2a bis 2e dargestellte Freigabeposition F bringbar. Die Träger bestehen aus hochtemperaturbeständigen thermoplastischen Kunststoffen wie etwa Polyetheretherketon (PEEK) oder Polyphenylsulfon (PPSU). Das Basiselement 5 hat die Dimensionen 10 mm x 6 mm x 26 mm; die Halterung hat die Dimensionen 6,2 mm x 5,5 mm x 18 mm.

In den Figuren 2a bis 2e ist der gleiche Träger 1 in der Freigabeposition F dargestellt. Durch die Ausübung einer Druckkraft in der ersten Richtung R1 wurde die Halterung 3 relativ zum Basiselement 5 bewegt. In dieser Freigabeposition F ist die Haltefläche 4 nicht mehr in Kontakt mit der Knochenschraube 2. In Folge dessen ist die Knochenschraube 2 in der zweiten Richtung R2 aus der Halterung 3 entnehmbar (siehe Figur 2d und 2e). Diese zweite Richtung R2 ist der ersten Richtung R1 entgegengesetzt.

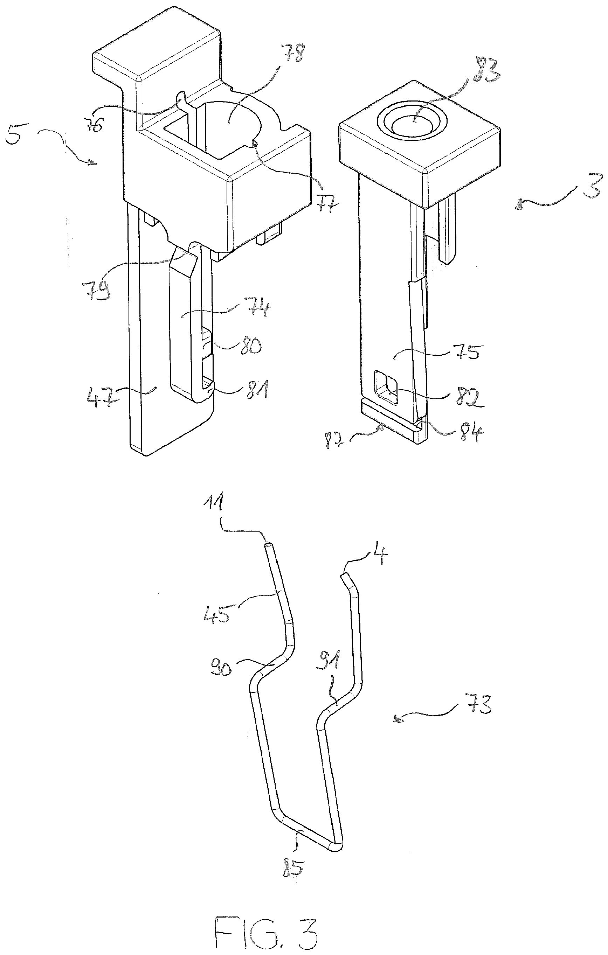

In der Figur 3 sind die Halterung 3, das Basiselement 5 sowie die als einstückiger Draht ausgebildeten Freigabemittel 73 getrennt dargestellt. Das Basiselement 5 weist eine Einschuböffnung 78 auf, in welche die Halterung 3 einschiebbar ist. An zwei gegenüberliegenden Seiten der Einschuböffnung 78 sind eine Durchbrechung 76 und eine Führungsnut 77 angeordnet. Am unteren Ende des Basiselements 5 ist eine erste Führungszunge 74 angeordnet, welche über Sollbruchmittel, die als materialgeschwächter Bereich 79 ausgebildet sind, mit dem Rest des Basiselements 5 verbunden ist. Am unteren Ende der ersten Führungszunge 74 sind eine erste Rastnase 80 und eine zweite Rastnase 81 angeordnet, welche im Wesentlichen senkrecht von der ersten Führungszunge 74 abstehen.

Die Halterung 3 weist eine Aufnahmeöffnung 83 für eine Knochenschraube auf. Am unteren Ende der Halterung 3 ist eine zweite Führungszunge 75 angeordnet. Am unteren Ende der zweiten Führungszunge 75 sind ein Rastloch 82 und eine Klemmnut 84 angeordnet.

Der Federdraht 73 weist an einem ersten Ende eine Rastzunge 45 auf, an deren Ende eine Führungsfläche 11 angeordnet ist. Am anderen Ende des Federdrahts 73 ist eine Haltefläche 4 für den Kontakt mit einer Knochenschraube angeordnet. Die beiden Schenkel des Federdrahts 73 weisen einen ersten Anschlagsbereich 90 und einen zweiten Anschlagsbereich 91 auf. Im unteren Bereich und damit in der Mitte des Federdrahts 73 befindet sich ein Klemmbereich 85. Bevorzugt ist der Federdraht 73 aus einem korrosionsbeständigen Stahl, wie beispielsweise Federstahl, aus Titan oder aus Nitinol gefertigt.

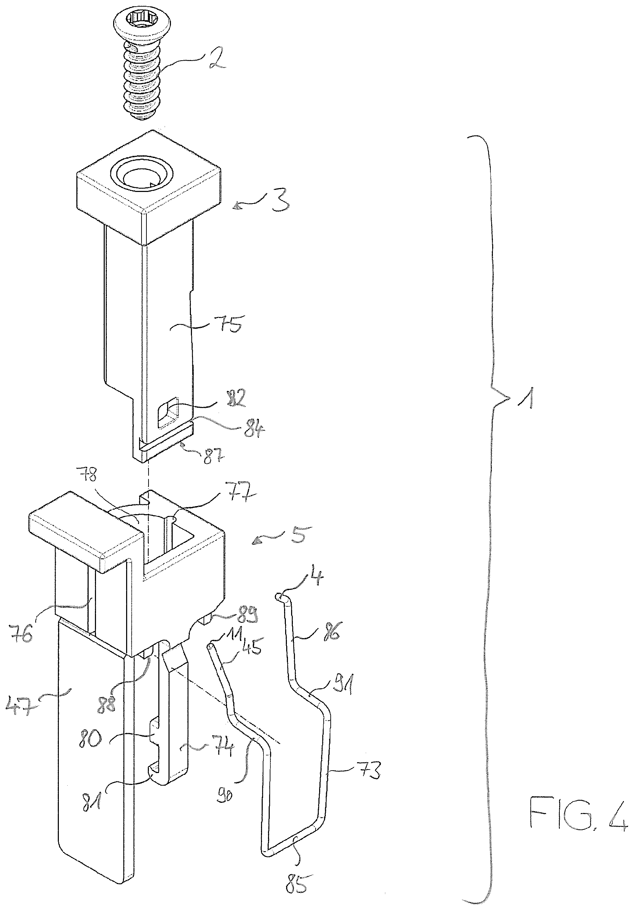

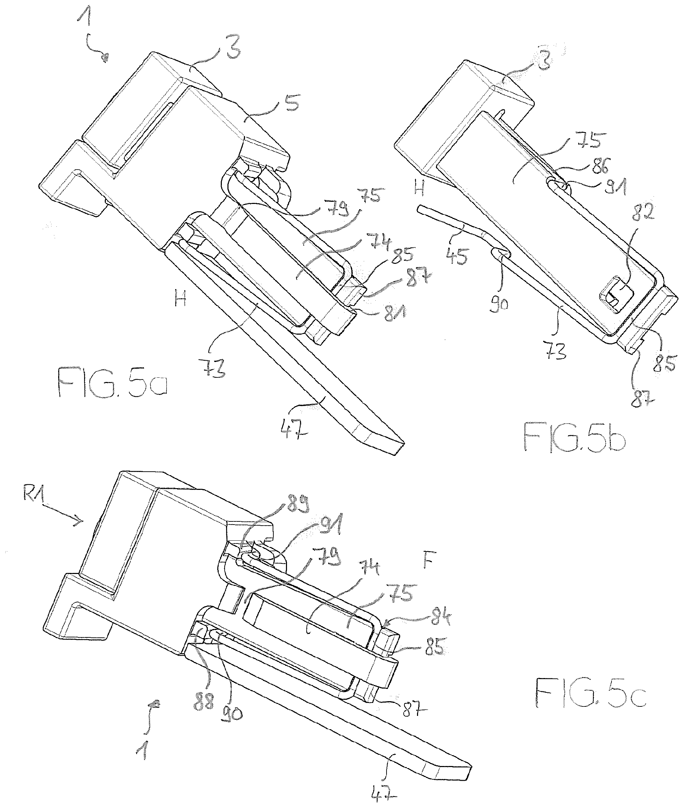

In der Figur 4 ist eine Explosionsdarstellung des erfindungsgemässen Trägers 1 dargestellt; die Figuren 5a bis 5c stellen den zusammengesetzten Träger 1 sowie insbesondere die Bewegung von der Halteposition H in die Freigabeposition F perspektivisch dar. Bei der Montage des Trägers 1 wird der Federdraht 73 in das Basiselement 5 eingesetzt. Dabei gerät der erste Anschlagsbereich 90 des Federdrahts 73 in seitlichen Kontakt mit einem ersten Nocken 88 am Basiselement 5. Weiterhin gerät der zweite Anschlagsbereich 91 des Federdrahts 73 in seitlichen Kontakt mit einem zweiten Nocken 89 am Basiselement 5. Ausserdem durchgreift die Rastzunge 45 die Durchbrechung 76 im Basiselement 5. Weiterhin gerät der seitliche Klemmbereich 86 vor die Führungsnut 77, dringt jedoch aufgrund des Anschlages des zweiten Anschlagsbereichs 91 am zweiten Nocken 89 nicht in diese Führungsnut 77 ein.

Zum Einsetzten der Halterung 3 in das Basiselement 5 wird die Halterung 3 in die Einschuböffnung 78 eingeschoben. Dabei greift die erste Rastnase 80 in das Rastloch 82 ein. Überdies gerät die Unterkante 87 der zweiten Führungszunge 75 in Kontakt mit der zweiten Rastnase 81. Weiterhin wird der untere Klemmbereich 85 des Federdrahts 73 mit der ersten Führungszunge in die Klemmnut 84 an der zweiten Führungszunge 75 eingeklemmt. Ferner gelangt die Haltefläche 4 am Ende des Federdrahts 73 in Kontakt mit der eingesetzten Knochenschraube 2.

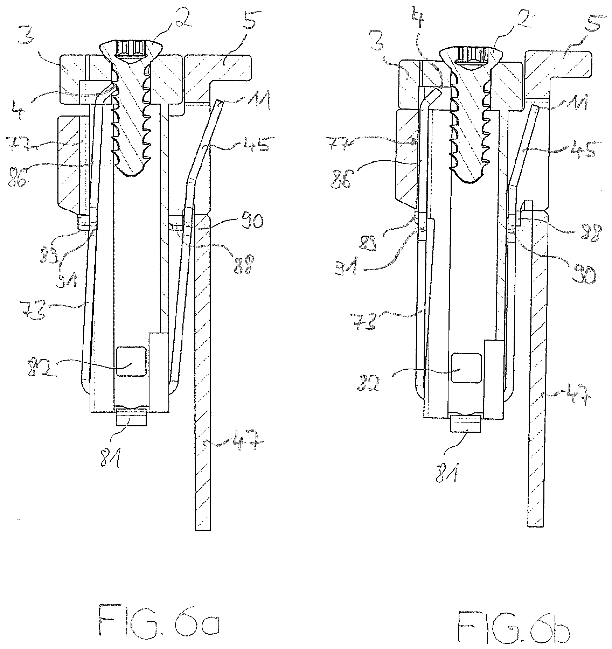

Beim Ausüben einer Druckkraft in der Richtung R1 wird der Träger von der Halteposition H in die Freigabeposition F überführt, welche in der Figur 5c dargestellt ist. Beim Ausüben der Drucckraft wird die zweite Führungszunge 75 in der Richtung R1 bewegt. Aufgrund der Klemmwirkung zwischen dem unteren Klemmbereich 85 und der Klemmnut 84 wird auch der Federdraht 73 in die Richtung R1 bewegt. Dadurch (siehe auch Querschnittdarstellung in den Figuren 6a und 6b) rutscht einerseits der erste Anschlagsbereich 90 am Federdraht 73 über den am Basiselement 5 angeordneten ersten Nocken 88. Hierdurch bewegt sich die Rastzunge 45 und insbesondere die Führungsfläche 11 in das Innere des Basiselements 5 hinein. Andererseits bewegt sich der zweite Anschlagsbereich 90 am Federdraht 73 über den zweiten Nocken 89 am Basiselement 5. Dies bewirkt, dass der seitliche Klemmbereich 86 des Federdrahts 73 in die Führungsnut 77 eindringt und die Haltefläche 4 von der Knochenschraube 2 wegbewegt wird. Somit vermittelt der Federdraht 73 sowohl das Einziehen der Rastzunge 45 und der Führungsfläche 11 als auch die Freigabe der Knochenschraube 2.

Aufgrund des Formschlusses zwischen der ersten Rastnase 80 am Basiselement 5 und dem Rastloch 82 an der Halterung 3 wird aufgrund der Bewegung der Halterung 3 auch die erste Führungszunge 74 des Basiselements 5 in der Richtung R1 gezogen. Auf diese Weise zerbricht der geschwächte Bereich 79. Zum Durchbrechen des geschwächten Bereichs 79 und damit auch zur Bewegung der Halterung 3 relativ zum Basiselement 5 ist somit eine minimale vorbestimmte Druckkraft erforderlich. Durch geeignete Ausbildung des geschwächten Bereich 79 ist es folglich möglich, die minimal erforderliche Druckkraft einzustellen, welche nötig ist, um die Halterung 3 relativ zum Basiselement 5 zu bewegen und damit den Träger 1 von der Halteposition H in die Freigabeposition F zu bewegen.

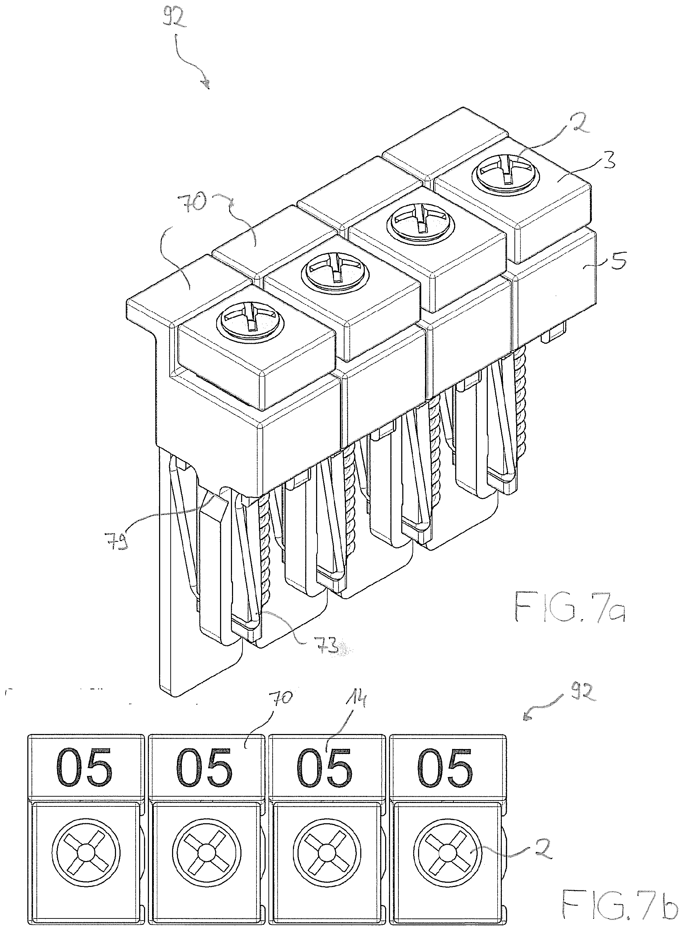

In den Figuren 7a und 7b ist eine Einheit 92 mehrerer erfindungsgemässer Träger 1 dargestellt. Die Träger 1 sind dabei über hier nicht dargestellte Verbindungsmittel miteinander fest oder lösbar verbunden. Die Verbindungsmittel können etwa eine Rastnase und eine komplementäre Aussparung, Sollbruchelemente oder eine nach dem Einsetzen entfernbare Haftfolie umfassen.

In Figur 7b ist eine Draufsicht auf die Einheit 92 dargestellt. Die Basiselemente 5 weisen Kennzeichnungsflächen 70 auf, auf denen Kennzeichnungen 14 wiedergegeben sind. Im dargestellten Beispiel ist z. B. die Länge der eingesetzten Knochenschraube 2 in Millimetern angegeben.

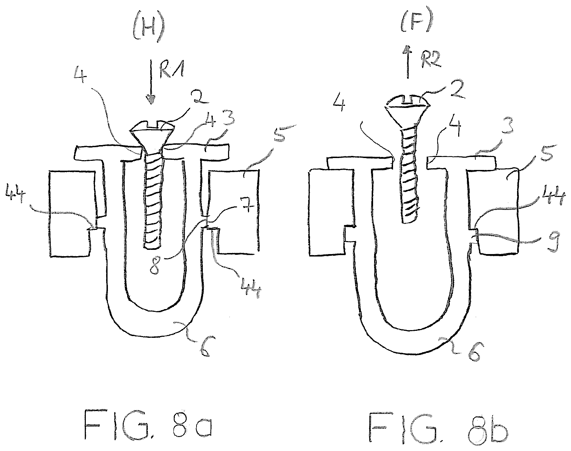

Eine alternative Ausführungsform des erfindungsgemässen Trägers 1 ist in den Figuren 8a und 8b dargestellt. Auch dieser Träger 1 enthält eine Halterung 3 und ein Basiselement 5. In der Figur 8a wird eine Knochenschraube 2 von zwei Halteflächen 4 in der Halterung 3 gehalten. Die beiden Halteflächen 4 sind an den Enden eines Federmittels 6 angeordnet. Mit Hilfe dieses Federmittels 6 ist die Halterung 3 am Basiselement 5 festgeklemmt. Die Klemmwirkung wirkt hierbei zwischen einer Kontaktfläche 7 an der Halterung 3 und einer Gegenkontaktfläche 8 am Basiselement 5. Bei Ausübung einer Kraft in der eingezeichneten Richtung R1 wird die Halterung 3 relativ zum Basiselement 5 bewegt. Die Kontaktfläche 7, welche als Oberfläche eines Nockens 9 ausgebildet ist, bewegt sich dabei über die Kante 44. In Folge der Vorspannung der Federmittel 6 wird nun die Halterung 3 in horizontaler Richtung auseinandergedrückt, so dass die Halteflächen 4 und die Knochenschraube 2 ausser Kontakt geraten (vgl. Figur 8b). Somit kann die Knochenschraube 2 in der Richtung R2, welche der Richtung R1 entgegengesetzt ist, entnommen werden.

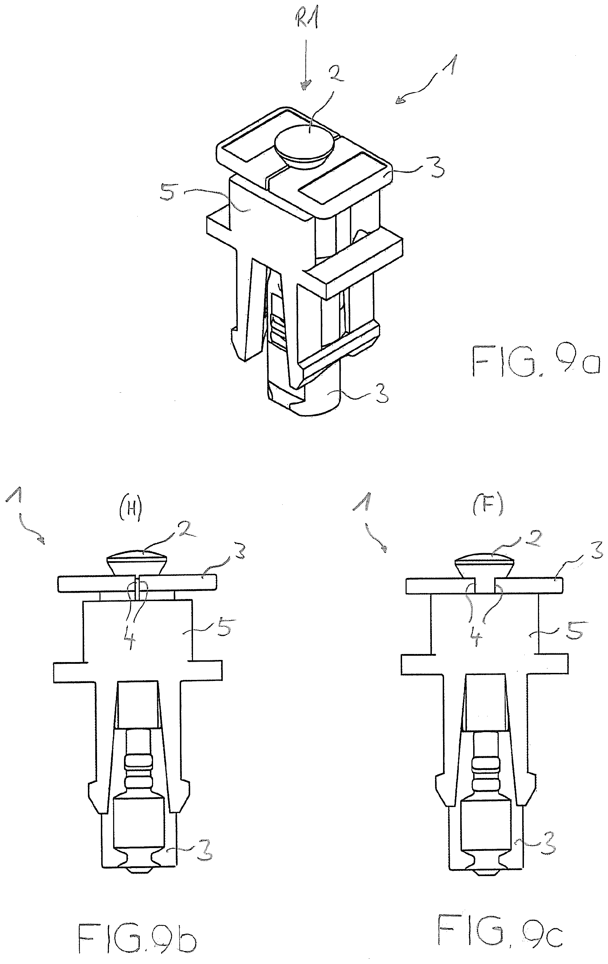

Eine weitere alternative Ausführungsform des erfindungsgemässen Trägers 1 ist in den Figuren 9a bis 9i dargestellt. Die Figur 9a zeigt eine perspektivische Darstellung eines solchen Trägers 1. Eine Knochenschraube 2 ist in einer Halterung 3 angeordnet, welche sich ihrerseits in einem Basiselement 5 befindet. In der Figur 9b ist eine Seitenansicht des Trägers 1 in einer Halteposition H dargestellt. In dieser Halteposition H wird die Knochenschraube 2 von den Halteflächen 4 gehalten. In der in Figur 9c dargestellten Freigabeposition F ist das Haltelement 3 im Vergleich zur Figur 4b relativ zum Basiselement 5 abgesenkt. In dieser Freigabeposition F sind die Halteflächen 4 nicht mehr in Kontakt mit der Knochenschraube 2. Somit wird die Knochenschraube 2 nicht mehr in der Halterung 3 gehalten und kann entnommen werden.

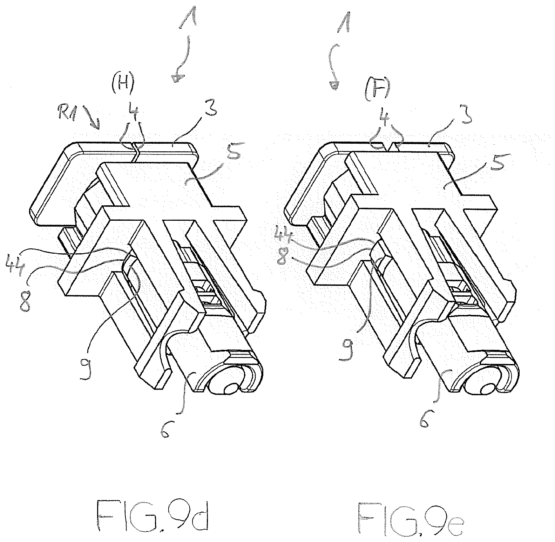

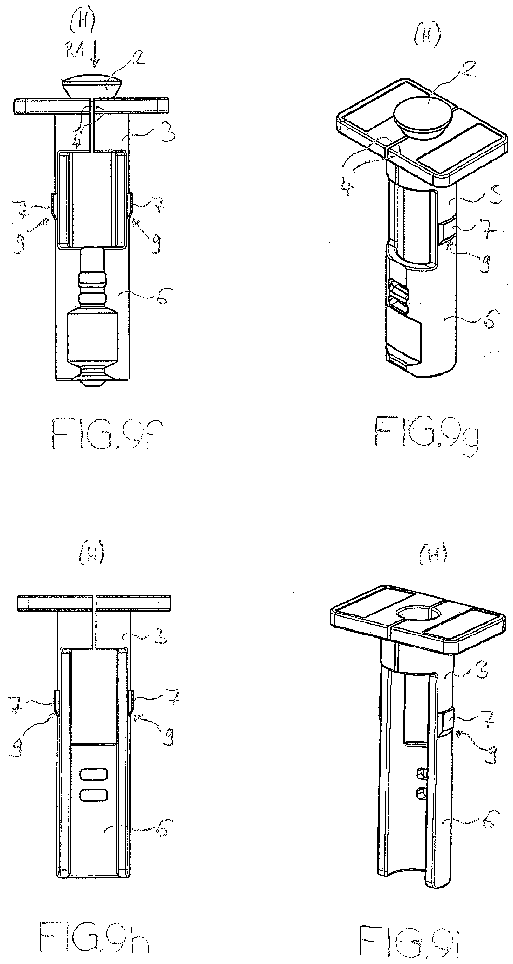

Die Figuren 9d und 9e zeigen zwei weitere perspektivische Ansichten des Trägers 1 in der Halteposition H bzw. in der Freigabeposition F. Die Federmittel 6 sind am unteren Ende der Halterung 3 angeordnet, welche in der in Figur 9d dargestellten Halteposition H derart vorgespannt sind, dass die beiden Halteflächen 4 auseinander gedrückt werden (siehe auch die separate Darstellung der Halterung 3 in den Figuren 9f bis 9i). An zwei gegenüberliegenden Seiten der Halterung 3 ist jeweils ein Nocken 9 angeordnet, von denen hier nur einer sichtbar ist. Die Oberflächen der Nocken 9 bilden Kontaktflächen 7, welche zumindest in der Halteposition H in Kontakt mit Gegenkontaktflächen 8 des Basiselements 5 sind. Bei der Ausübung einer Druckkraft in der Richtung R1 wird die Halterung 3 gegenüber dem Basiselement 5 abgesenkt. Die Kontaktfläche 7 bewegt sich dabei über eine Kante 44 am Basiselement 5. Infolge der Vorspannung der Federmittel 6 wird nun die Halterung 3 in horizontaler Richtung auseinander gedrückt, sodass die Halteflächen 4 und die Knochenschraube 2 ausser Kontakt geraten.

Die Figuren 9f bis 9i zeigen separat das Halteelement 3 in verschiedenen Ansichten. Das Halteelement ist jeweils in der Halteposition, wobei das Basiselement 5 hier jeweils nicht dargestellt ist. Dabei zeigt die Figur 9f eine Frontansicht und die Figur 9h eine Rückansicht, während die Figuren 9g und 9i perspektivische Darstellungen zeigen. Das untere Ende der Halterung 3 fungiert hier als Federmittel 6, welches in der in den Figuren 9f bis 9i dargestellten Halteposition H derart vorgespannt ist, dass die beiden Halteflächen 4 auseinandergedrückt werden. An zwei gegenüberliegenden Seiten der Halterung 3 ist jeweils ein Nocken 9 angeordnet. Die Oberflächen der Nocken 9 bilden Kontaktflächen 7, welche zumindest in der Halteposition H in Kontakt mit hier nicht dargestellten Gegenkontaktflächen 8 des Basiselements 5 sind. Bei der Ausübung einer Druckkraft in der in Figur 9a dargestellten Richtung R1 wird die Halterung 3 gegenüber dem Basiselement abgesenkt. Die Kontaktfläche 7 bewegt sich dabei über eine Kante 44 am Basiselement 5. In Folge der Vorspannung der Federmittel 6 wird nun die Halterung 3 in horizontaler Richtung auseinandergedrückt, so dass die Halteflächen 4 und die Knochenschraube 2 ausser Kontakt geraten.

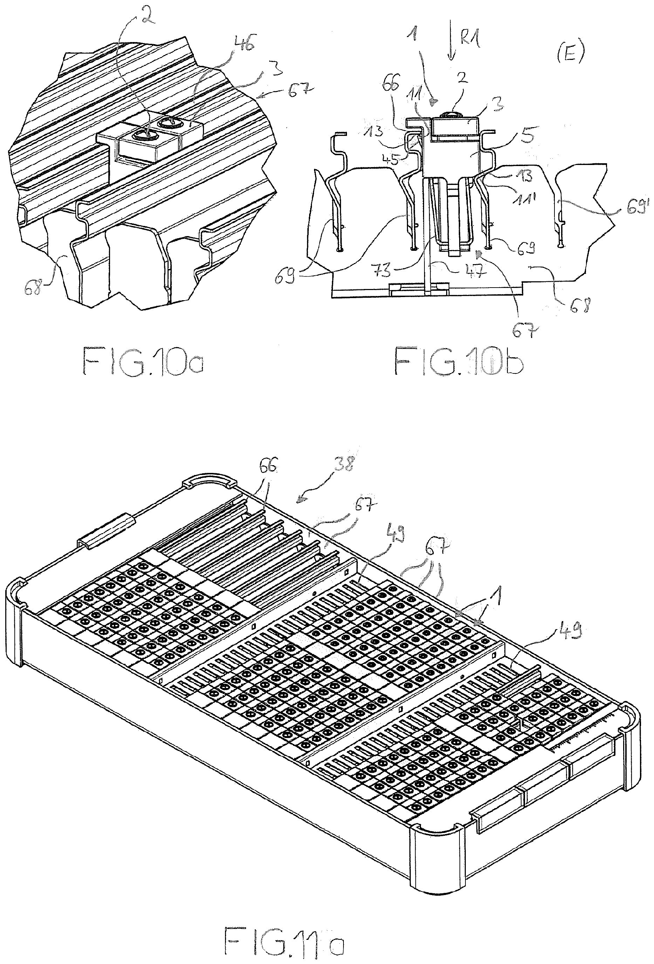

In den Abbildungen 10a und 10b sind Träger 1 dargestellt, welche in den Zwischenraum 67 zwischen zwei benachbarten Führungslamellen 66 eingesetzt sind. Jede der Führungslamellen 66 ist in ein Paar beabstandeter Lamellenaufnahmen 69 eingesetzt, welche als Schlitze in zwei beabstandeten Trennwänden 68 eines chirurgischen Behälters ausgebildet sind. In beiden Figuren ist jeweils nur eine der Trennwände 68 eingezeichnet. Dabei können einige oder alle Führungslamellen 66 lösbar mit den Trennwänden 68 verbunden sein. Somit ist es möglich, einige oder mehrere der Führungslamellen 66 zu entfernen und auf diese Weise einen Freiraum zu schaffen, in welchen etwa ein Aufnahmefach für chirurgische Instrumente einsetzbar ist. In der Figur 10b ist beispielsweise in der Lamellenaufnahme 69' kein Führungslamelle 66 eingesetzt.

Die Träger 1 weisen Führungsflächen 11,11' auf, welche mit den Gleitflächen 13 der Führungslamellen 66 in Kontakt sind und relativ zu diesen verschiebbar sind. Eine der Führungsflächen 11 des Trägers 1 ist am Ende einer Rastzunge 45 angeordnet, während die hierzu komplementäre Gleitfläche 13 am oberen Ende der Führungslamelle 66 angeordnet ist. Eine zweite Führungsfläche 11' ist am Basiselement 5 des Trägers 1 angeordnet. Beim Einsetzen des Trägers 1 in einen Zwischenraum 67 rastet die an der Halterung 3 angeordnete Rastzunge 45 unter die Gleitfläche und verbindet somit den Träger 1 mit den Führungslamellen 66. Somit befindet sich der Träger 1 in einer Einrastposition E.

Bei der Ausübung einer Kraft in der Richtung R1 wird die Halterung 3 von der Halteposition H in die Freigabeposition F gebracht (vgl. Figuren 1a bis 1e sowie 2a bis 2e). Der Federdraht 73 bewirkt, dass gleichzeitig die Rastzunge 45 derart bewegt, dass sie den Kontakt mit der Führungslamelle 11 verliert. Auf diese Weise wird der Träger 1 in eine hier nicht dargestellte Entnahmeposition M überführt, in welcher er aus dem Zwischenraum 67 entfernt werden kann.

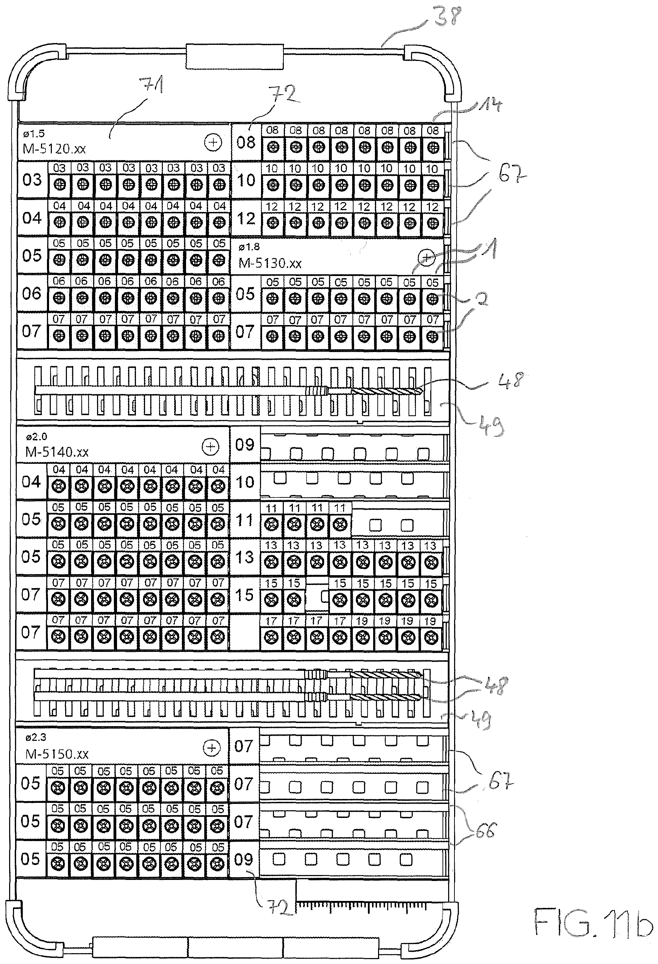

In den Figuren 11a und 11b ist ein chirurgischer Behälter 38 dargestellt, welcher mehrere Führungslamellen 66 aufweist. Die perspektivische Ansicht 11a zeigt mehrere in die Zwischenräume 67 zwischen den Lamellen eingesetzte Träger 1. Der modulare Aufbau gestattet es, nach Belieben eine Auswahl von Trägern 1 mit darin aufgenommenen Knochenschrauben 2 in die Zwischenräume 67 einzusetzen.

Weiterhin enthält der Behälter 38 Aufnahmefächer 49 für hier nicht dargestellte chirurgische Instrumente. Die Aufnahmefäeher 49 sind derart ausgebildet und bemessen, dass sie ebenfalls mit den Führungslamellen 66 einrastbar in Kontakt bringbar sind. Insbesondere sind die Aufnahmefächer 49 derart bemessen, dass sie mit den Abständen der Lamellenaufnahmen 69 und damit der Führungslamellen 66 kompatibel sind. Dabei ist es durch eine oben beschriebene Entnahme einer oder mehrerer Führungslamellen 66 möglich, dass ein Aufnahmefach 49 eine Ausdehnung hat, welche einem ganzzahligen Vielfachen des Abstandes zweier Lamellenaufnahmen 69 entspricht.

Figur 11b zeigt in der Draufsicht den gleichen chirurgischen Behälter 38. Dieser enthält mehrere Führungslamellen 66. In die Zwischenräume 67 sind jeweils mehrere Träger 1 mit Knochenschrauben 2 eingesetzt sind. Die Träger 1 weisen Kennzeichnungen 14 auf, welche auf einer nach aussen sichtbaren Kennzeichnungsflächen 70 Produktinformationen (in diesem Falle die Längen der Schrauben in Millimetern) zeigen.

Überdies sind in dem Behälter 38 Gruppenmarkierungsfelder 71 angeordnet, welche zwischen den Führungslamellen 66 lösbar eingerastet sind und gemeinsame Kennzeichnungen einer Gruppe von chirurgischen Materialien aufweisen. Diese Kennzeichen können beispielsweise Schraubendurchmesser, Produktnummern oder sonstige technische Spezifikationen wie die Form des Schraubenkopfes enthalten. Weiterhin sind in dem Behälter 38 Zeilenmarkierungsfelder 72 angeordnet, welche ebenfalls zwischen den Führungslamellen 66 lösbar eingerastet sind und Kennzeichnungen einer Reihe von chirurgischen Materialien aufweisen, die etwa neben den Zeilenmarkierungsfeldern 72 angeordnet sind.

Insgesamt weist der chirurgische Behälter 38 somit ein hohes Mass an Modularität auf, da er mit den einzelnen Komponenten nach Bedarf bestückbar ist. So ist etwa die Auswahl und Anzahl von Knochenschrauben 2 und an chirurgischen Instrumenten 49 lediglich durch die Gesamtgrösse des Behälters 38 beschränkt. Somit ergibt sich für den Benutzer gleichzeitig ein Höchstmass an Individualität und Übersichtlichkeit.

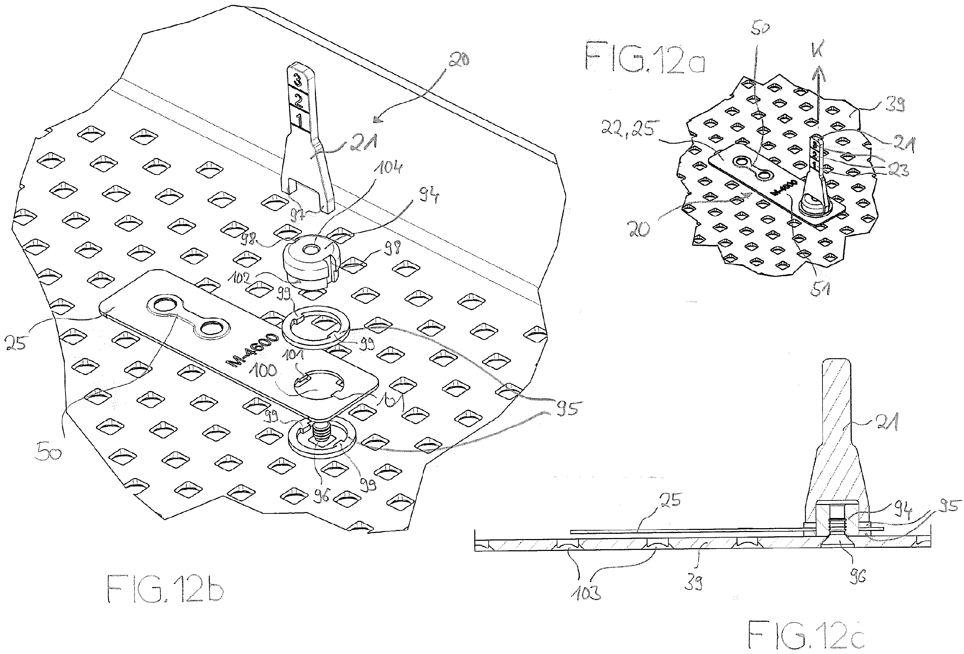

In den Figuren 12a bis 12c ist eine erfindungsgemässe Halterung 20 zum Fixieren wenigstens eines hier nicht dargestellten Knochenplattenträgers für eine Knochenplatte dargestellt. Die Halterung 20 ist mit Hilfe von Befestigungsmitteln an einer gelochten Bodenplatte 39 befestigt. Die Halterung 20 weist einen Steg 21 auf, der entlang einer Kennzeichenrichtung K mit Kennzeichnungen 23 in Form einer Skala versehen ist. Diese Skala enthält die natürlichen Zahlen von 1 bis 3. Die Halterung 20 weist ferner ein flächiges Formelement 25 auf, welches mit der gedachten Grundebene 22 übereinstimmt. Die Zahlenangaben auf der Skala stehen im Sinne der Erfindung in eindeutigem Zusammenhang zu dem Abstand von dieser Grundebene 22.

Das Formelement 25 besteht aus Metall oder aus Kunststoff wie etwa einem hochtemperaturbeständigen thermoplastischen Kunststoffe wie Polyetheretherketon (PEEK) oder Polyphenylsulfon (PPSU). Es wird beispielsweise durch Laserschneiden, Wasserstrahlschneiden oder mechanisches Schneiden mit Messern hergestellt. Bevorzugt erfolgt die Herstellung mittels Laserstrahlschneiden von Metallblech. Ferner gibt das Formelement 25 die Form 50 einer Knochenplatte wieder. Darüber hinaus enthält das Formelement 25 eine Spezifikation 51, welche beispielsweise die Produktnummer einer Knochenplatte oder Herstellerangaben darstellen kann. Die Spezifikation kann beispielsweise durch eine Laserbeschriftung aufgebracht sein.

Die Art der Befestigung der Halterung 20 mit Hilfe der Befestigungsmittel ist in den Figuren 12b und 12c genauer dargestellt.

Die Befestigungsmittel umfassen einen Haltesockel 94, zwei Distanzringe 95 sowie eine Halteschraube 96. Das Formelement 25 weist eine im Wesentlichen kreisförmige Öffnung 100 auf, in die sich zwei Formelementvorsprünge 101 erstrecken. Die beiden Distanzringe 95 weisen jeweils zwei Ringvorsprünge 99 auf. Das Formelement 25 ist zwischen den beiden Distanzringen 95 angeordnet, wobei die Ringvorsprünge 99 und die Formelementvorsprünge 101 aufeinander ausgerichtet sind. Der Haltesockel 94 durchdringt das Formelement 25 und die beiden Distanzringe 95, wobei zwei Sockelnuten 98 in Eingriff mit den Ringvorsprüngen 99 und den Formelementvorsprüngen 101 gelangen. Eine Anschlag-Kante 102 des Haltesockels 94 liegt dabei auf dem oberen Distanzring 95 auf. Der Steg 21 enthält zwei Stegvorsprünge 97, welche ebenfalls mit den beiden Sockelnuten 98 des Haltesockels 94 in Eingriff gebracht sind.

Zum Fixieren des Steges 21, des Haltesockels 94, des Formelements 25 und der beiden Distanzringe 95 auf einer Bodenplatte 39 werden diese Bauteile über eine Öffnung 103 in der Bodenplatte 39 gesetzt. Von der Unterseite der Bodenplatte 39 wird die Halteschraube 96 durch die Öffnung 103 (siehe Figur 12c) in der Bodenplatte 39, durch die beiden Distanzringe 95 und durch die Öffnung 100 im Formelement 25 geführt und in einer axialen Bohrung 104 des Haltesockels 94 fixiert. Der Kontakt der Kante 102 am Haltesockel 94 mit dem oberen Distanzring 95 bewirkt somit eine feste Einspannung des Formelements 25 relativ zur Bodenplatte 39. Der Distanzring 95 bewirkt auch, dass die untere Kontaktfläche 29 nicht auf dem Formelement 25 aufliegt und diese einer Sterilisation zugänglich ist. Aufgrund des unteren Distanzrings 95 ist das Formelement 25 von der Bodenplatte 39 beabstandet angeordnet (vgl. Figur 12c), sodass auch die Unterseite des Formelements 25 einer Sterilisation zugänglich ist. Weiterhin wird durch das Einsetzen der Halteschraube 96 in den Haltesockel 94, der Haltesockel 94 auseinander gespreizt, sodass mittels des Kontakts zwischen den Stegvorsprüngen 97 und den Sockelnuten 98 auch der Steg 21 relativ zum Formelement 25 und der Bodenplatte 39 fixiert ist. Die Stegvorsprünge 97 werden, nachdem sie in die Sockelnuten 98 eingesetzt sind, mit diesen verschweisst oder verklebt. Der Steg 21 und der Haltesockel 94 können auch aus einem Stück hergestellt werden. Bevorzugt bestehen die Bauteile der Halterung 20 aus rostfreiem Stahl oder hochtemperaturbeständigen thermoplastischen Kunststoffen wie Polyetheretherketon (PEEK) oder Polyphenylsulfon (PPSU).

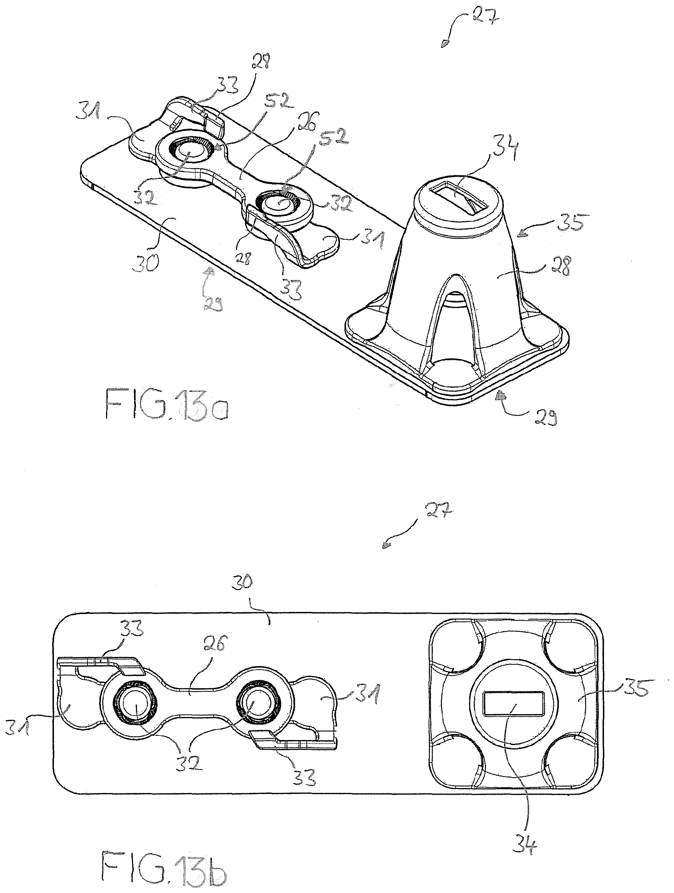

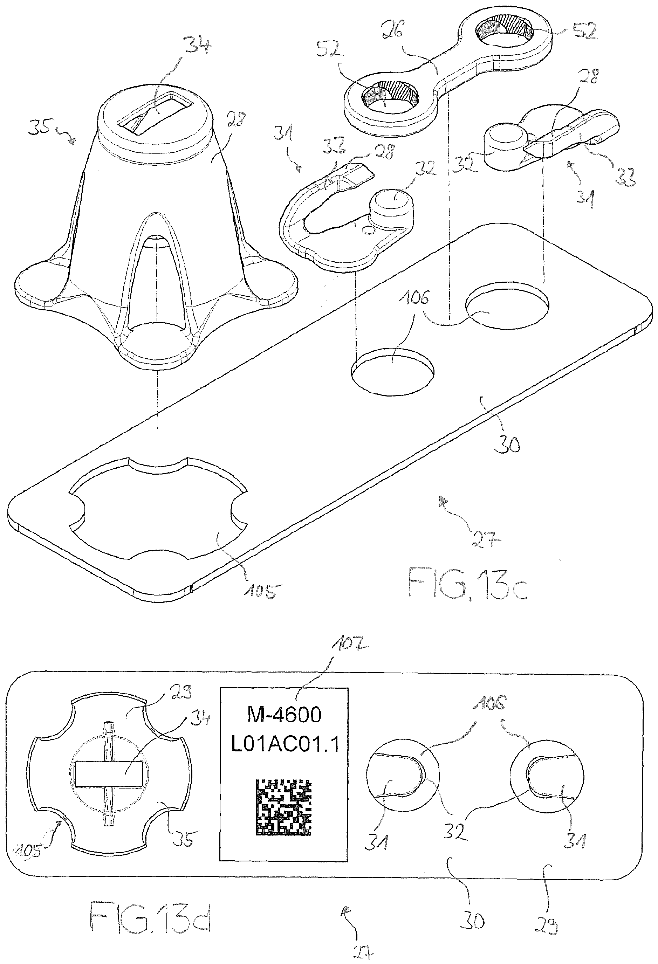

In den Figuren 13a bis 13d ist ein erfindungsgemässer Knochenplattenträger 27 mit einer Knochenplatte 26 dargestellt. Der Knochenplattenträger 27 weist eine Grundfläche 30 auf, welche aus einem hochtemperaturbeständigen thermoplastischen Kunststoff wie Polyetheretherketon (PEEK) oder Polyphenylsulfon (PPSU) besteht. Auf der Grundfläche 30 sind zwei Halteelemente 31 angeordnet. Diese Halteelemente 31 bestehen ebenfalls aus einem hochtemperaturbeständigen thermoplastischen Kunststoff wie Polyetheretherketon (PEEK) oder Polyphenylsulfon (PPSU) und sind zumindest teilweise elastisch ausgebildet. Die Halteelemente 31 werden bevorzugt durch Kleben auf der Grundfläche 30 fixiert. Bevorzugt werden die Teile durch Ultraschall miteinander verschweisst. Kleben ist auch denkbar. Beide Halteelemente 31 weisen jeweils einen Stützbereich 32 in Form eines zentralen Kegelstumpfes sowie seitliche Schnappelemente 33 auf. Die Haltelemente 31 sind jeweils an einer Halteelementdurchbrechung 106 (siehe Figur 13c) in der Grundfläche angeordnet, sodass die Stützbereiche 32 jeweils über einer Halteelementdurchbrechung 106 angeordnet sind.

Auf den Halteelementen 31 ist eine Knochenplatte 26 fixiert. Dabei sind die Halteelemente 31 und die Knochenplatte 26 derart aufeinander abgestimmt, dass die Öffnungen 52 der Knochenplatte 26 auf den Stützbereichen 32 liegen und die Knochenplatte 26 von den Schnappelementen 33 seitlich gehalten und in Richtung der Grundfläche 30 gedrückt wird. Die Knochenplatte 26 ist so mit den Halteelementen 31 in Kontakt, dass kein Kontakt zwischen der Knochenplatte 26 und der Grundfläche 30 besteht.

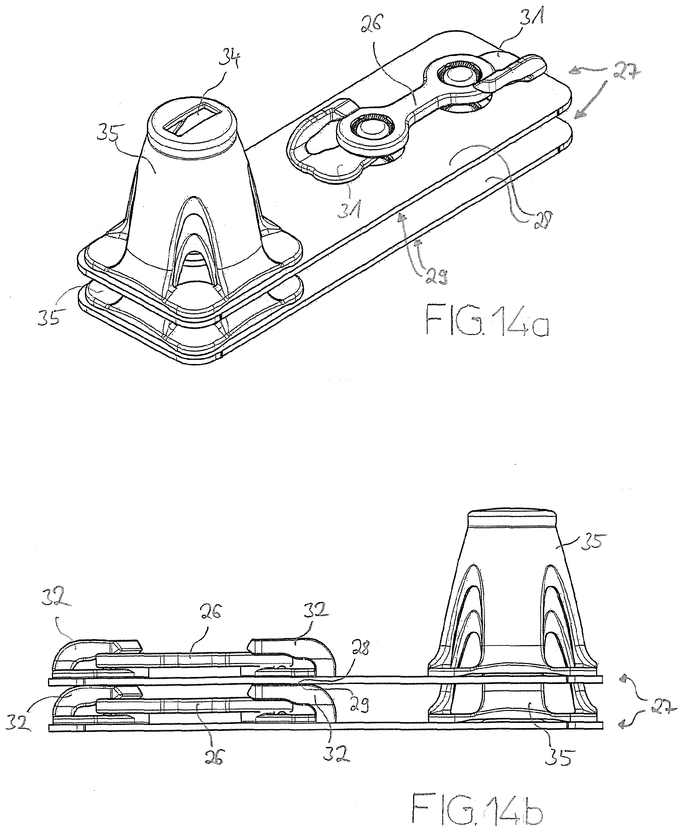

Weiterhin weist der Knochenplattenträger 27 ein Griffelement 35 auf, welches im Wesentlichen die Form eines hohlen Kegelstumpfes hat. Das Griffelement 35 ist von einer Öffnung 34 durchdrungen. Das Griffelement 35 besteht ebenso aus einem hochtemperaturbeständigen thermoplastischen Kunststoff wie Polyetheretherketon (PEEK) oder Polyphenylsulfon (PPSU). Auch die Griffelemente 35 sind bevorzugt auf die Grundfläche 30 aufgeklebt. Hier ist ebenfalls Ultraschallschweissen vorgesehen. Die Oberseite des Griffelements 35 sowie die Oberseiten der Schnappelemente 32 bilden obere Kontaktflächen 28, welche das Stapeln mehrerer Knochenplattenträger 27 erlauben (vgl. Figuren 14a und 14b sowie die zugehörige Beschreibung weiter unten).

Die Figur 13d zeigt eine Untenansicht des Knochenplattenträgers 27. Durch die Griffdurchbrechung 105 ist die innere Oberfläche des hohlen Griffelements 35 zu erkennen. Diese innere Oberfläche bildet einen Teil der unteren Kontaktflächen 29, welche das Stapeln mehrerer Knochenplattenträger 27 erlauben. Weiterhin sind durch die Halteelementdurchbrechungen 106 Teile der Halteelemente 31 und insbesondere der kegelstumpfförmigen Stützbereiche 32 sichtbar. Die Anordnung der Stützbereiche 32 über diesen Halteelementdurchbrechungen 106 ermöglicht ein ideales Sterilisieren einer Knochenplatte 26 auch an deren Unterseite. Die Unterseite der Grundfläche 30 bildet einen weiteren Teil der unteren Kontaktfläche 29, mittels deren mehrere der Knochenplattenträger 27 gestapelt werden können. Diese Unterseite weist Kennzeichnungen auf, welche hier eine Produktnummer, eine Seriennummer und einen maschinenlesbaren Code umfassen. Diese Kennzeichnungen werden bevorzug mit Hilfe einer Laserbeschriftung angebracht. Ein Druckverfahren wie Tampondruck oder Siebdruck ist aber auch denkbar.

Die Knochenplattenträger 27 und insbesondere die Griffelemente 35 sowie die Halteelemente 31 sind derart ausgebildet, dass mehrere der Knochenplattenträger 27 übereinander stapelbar sind, wie in den Figuren 14a und 14b dargestellt ist. Die Oberseiten der Griffelemente 35 sowie die Oberseiten der Schnappelemente 32 und die äusseren Oberflächen des Griffelements 35 bilden jeweils obere Kontaktflächen 28, während die inneren Oberflächen der hohlen Griffelemente 35 und die Unterseiten der Grundflächen 30 untere Kontaktflächen 29 bilden. Bei den gestapelten Knochenplattenträgern 27 sind die oberen Kontaktflächen 28 mit den unteren Kontaktflächen 29 des jeweils darüber angeordneten Knochenplattenträgers 27 in Kontakt (siehe Figur 14a und 14b). Allerdings besteht kein Kontakt zwischen einer Knochenplatte 26 und der Grundfläche 30 des darüber angeordneten Knochenplattenträgers 27. Auf Grund dieser geringen Kontaktflächen steht eine grosse freie Oberfläche der Knochenplatten 26 zur Verfügung, welche der Sterilisation frei zugänglich ist.

Die Öffnungen 34 sind derart im Griffelement 35 angeordnet, dass die Öffnungen 34 jeweils benachbarter Knochenplattenträger 27 zueinander fluchtend ausgerichtet sind, wenn mehrere der Knochenplattenträger 27 übereinander gestapelt sind.

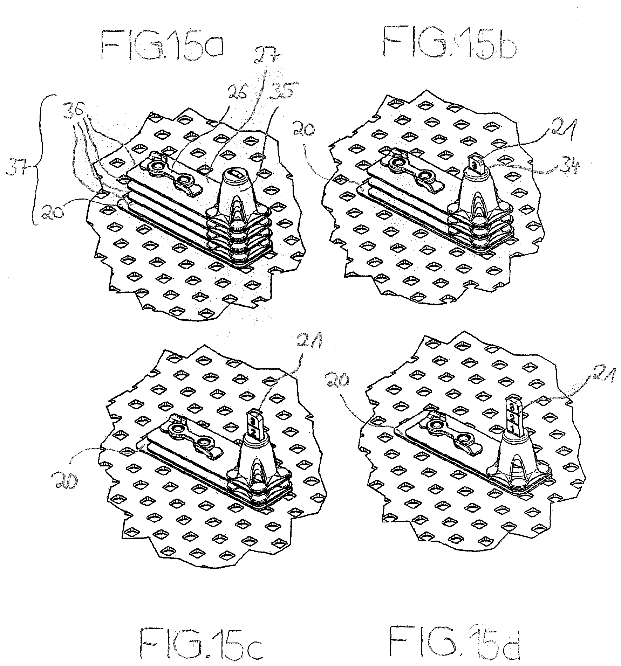

In den Abbildungen 15a bis 15d ist ein System 37 aus einer Halterung 20 zum Fixieren und mit vier, drei, zwei bzw. einer Einheit 36 dargestellt. Jede der Einheiten 36 umfasst einen Knochenplattenträger 27 sowie eine Knochenplatte 26. Dabei sind der Steg 21 und die Griffelemente 35 derart aufeinander abgestimmt, dass mehrere der Knochenplattenträger 27 derart übereinander stapelbar sind, dass die übereinander gestapelten Knochenplattenträger 27 gemeinsam auf den Steg 21 aufsetzbar sind und in den Figuren 9a bis 9d aufgesetzt sind. Der Steg 21 durchdringt dabei die zueinander fluchtend ausgerichteten Öffnungen 34 der Knochenplattenträger 27. Die in den Figuren 9a bis 9d dargestellte Anordnung ermöglicht es, mit einem Blick festzustellen, wie viele Einheiten 36 aus Knochenplattenträger 27 und Knochenplatte 26 sich auf der Halterung 20 befinden. So ist etwa gemäss Figur 9b der Skala auf dem Steg 21 zu entnehmen, dass noch genau drei solcher Einheiten 36 vorhanden sind. Dies vereinfacht eine Inventur und ein allfälliges Nachbestellen der Einheiten 36 erheblich. Alternativ zu der hier dargestellten Ausführungsform ist es denkbar, dass die Skala in der umgekehrten Richtung angeordnet ist. Auf diese Weise lässt sich beispielsweise ablesen, wie viele Knochenplatten 26 einem Knochenträger 27 entnommen wurden und demnach nachbestellt werden müssten.

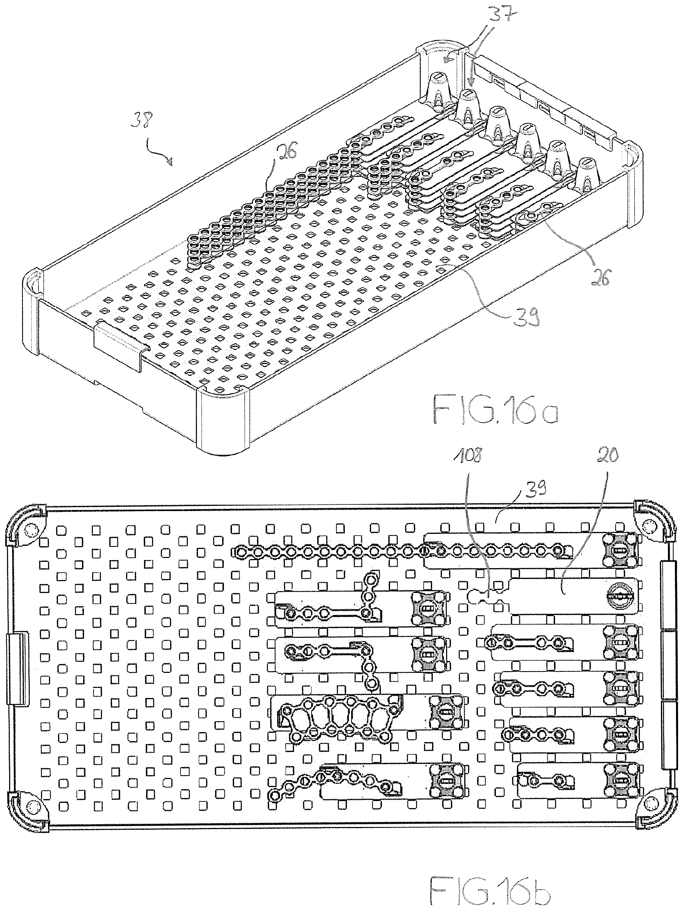

In den Figuren 16a und 16b sind mehrere Systeme 37 aus jeweils einer Halterung 20 und mehreren Einheiten 36 dargestellt, welche auf einer gelochten Bodenplatte 39 in einem chirurgischen Behälter 38 fixiert sind. Der modulare Aufbau erlaubt es, die Bodenplatte 39 nach Bedarf mit einem oder mehreren Systemen 37 mit Knochenplattenträgern 27 zu bestücken, welche die gewünschten Knochenplatten 26 tragen. Diese Darstellung zeigt ebenso, dass Stützbereiche 32 der Halteelemente 31 nicht notwendigerweise in direkt benachbarte Öffnungen 52 in einer Knochenplatte 26 eingreifen müssen. Beispielsweise wird die oberste in Figur 16b dargestellte Knochenplatte 26 an zwei Öffnungen 52 gehalten, zwischen welchen sich sechs kontaktfreie Öffnungen 52 befinden. Weiterhin zeigt Figur 16b auch eine Halterung 20, auf welcher sich momentan kein Knochenplattenträger befindet. Diese Halterung 20 weist überdies einen Kennzeichnungsfortsatz 108 auf, welcher die Form einer für diese Halterung 20 vorgesehenen Knochenplatte wiedergibt.

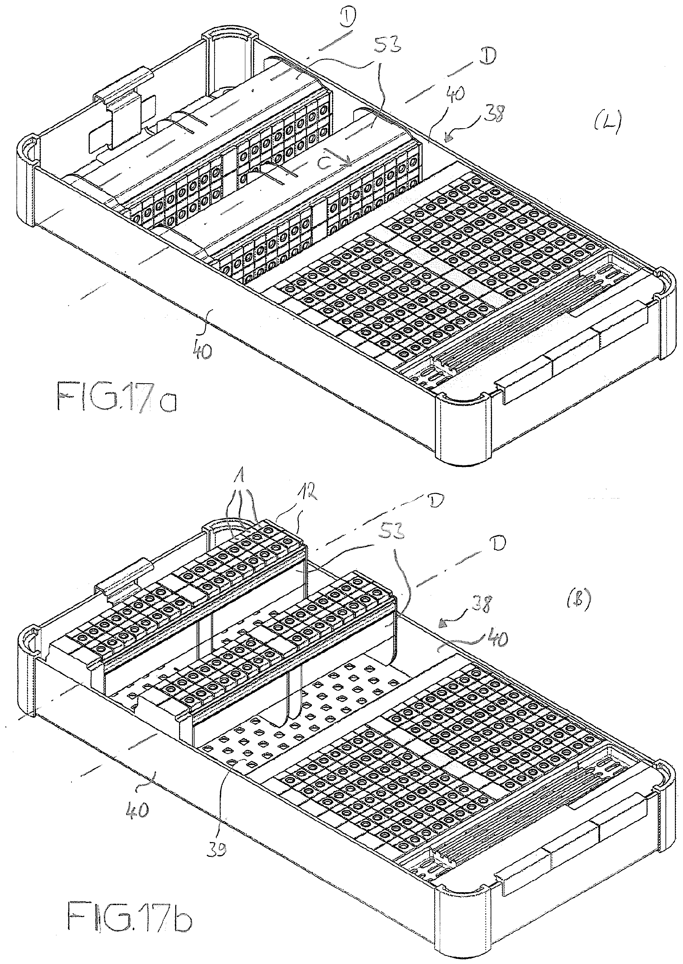

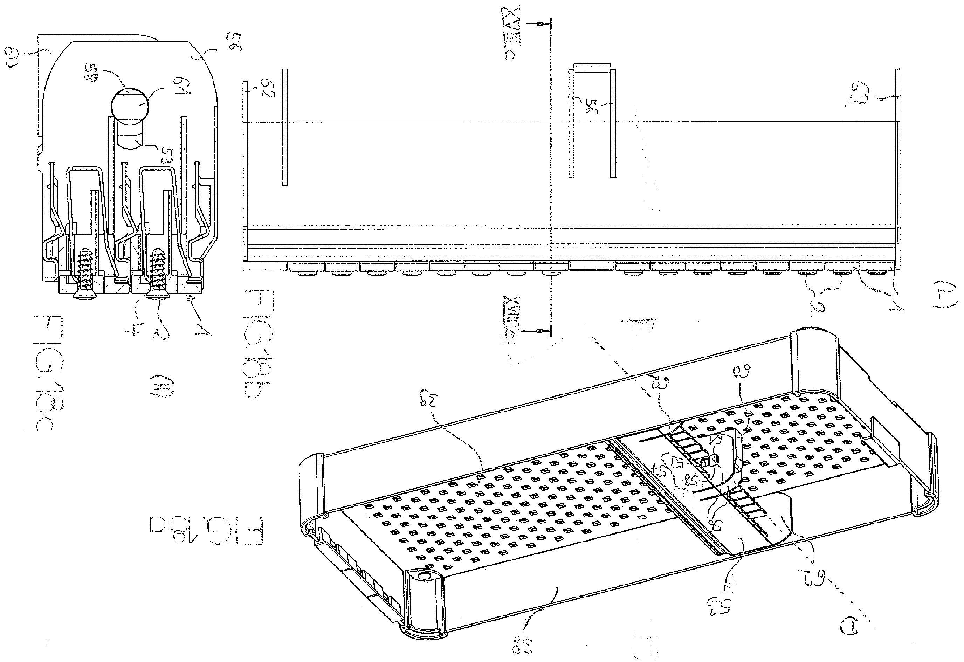

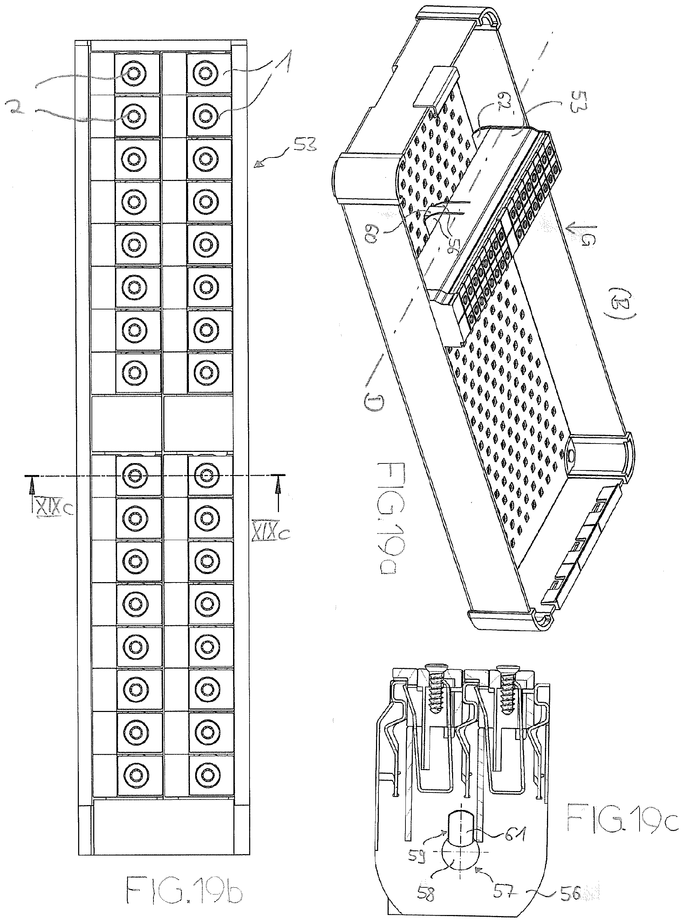

In den Figuren 17a und 17b ist ein chirurgischer Behälter 38 mit zwei erfindungsgemässen Aufnahmemodulen 53 dargestellt. Die Aufnahmemodule 53 sind mit Hilfe von in den Figuren 18a bis 18c sowie 19a bis 19c dargestellten Drehverbindungsmitteln an dem Behälter 38 fixiert. Die Aufnahmemodule 53 sind jeweils um eine Drehachse D relativ zum Behälter 38 drehbar. Die Drehachsen D sind parallel zur Bodenplatte 39 des Behälters 38 angeordnet. Jedes der beiden Aufnahmemodule 53 verfügt über Führungslamellen 66. In die Zwischenräume 67 sind jeweils mehrere erfindungsgemässe Träger einsetzbar sind. Zur Vereinfachung der Illustration sind in dieser Abbildung keine Knochenschrauben dargestellt.

In der in Figur 17a dargestellten Lagerungsposition L sind die Knochenschrauben 2 liegend angeordnet. Die Hauptausdehnungsrichtung C der Knochenschraube 2, welche mit der axialen Richtung der Knochenschrauben 2 übereinstimmt, liegt somit parallel zu der Bodenplatte 39. Diese Bauweise ermöglicht die Lagerung von Knochenschrauben 2 mit einer Länge, welche die Höhe des Behälters 38 übersteigt.

In der Figur 17b ist der gleiche Behälter 38 dargestellt, wobei sich jedoch beide Aufnahmemodule 53 in einer Bereitstellungsposition B befinden. In dieser Bereitstellungsposition B sind die Knochenschrauben 2 vertikal angeordnet und können daher leicht nach oben entnommen werden.

Die Figuren 18a bis 18c sowie 19a bis 19c verdeutlichen die Drehverbindungsmittel sowie deren Funktion. Dabei zeigt jeweils die Figur 18a bzw. 19a eine perspektivische Ansicht eines Behälters 38 mit einem Aufnahmemodul 53, die Figur 18b bzw. 19b zeigt jeweils eine Draufsicht auf das Aufnahmemodul 53, und die Figur 18c bzw. 19c zeigt jeweils eine Schnittzeichnung. Das Aufnahmemodul 53 weist zwei Haltelaschen 56 auf, in denen jeweils ein Eingriffloch 57 angeordnet ist. Jedes der Eingrifflöcher 57 verfügt über einen kreisförmigen Abschnitt 58 und über einen Langlochabschnitt 59. An der Bodenplatte 39 des Behälters 38 ist ein Haltesockel 60 angeordnet, welcher an zwei gegenüberliegenden Seiten über jeweils einen Eingriffsvorsprung 61 verfügt. Der Querschnitt des Eingriffsvorsprungs 61 besitzt die Form eines an zwei gegenüberliegenden Seiten um ein Kreissegment verringerten Kreises. Die Eingriffsvorsprünge 61 sind lösbar oder unlösbar mit den Eingrifflöchern 57 in Eingriff gebracht. Weiterhin verfügt das Aufnahmemodul 53 über zwei Stützlaschen 62, welche dem Aufnahmemodul 53 in der Bereitstellungsposition B zusätzlichen Halt geben.

In der in den Figuren 18a bis 18c dargestellten Lagerungsposition L sind die Eingriffsvorsprünge 61 in den kreisförmigen Abschnitten 58 der Eingriffslöcher 57 angeordnet. Gemäss Figur 18c sind jeweils die Halteflächen 4 der Träger 1 in Kontakt mit den eingesetzten Knochenschrauben 2. Folglich befinden sich die beiden hier dargestellten Träger 1 in einer Halteposition H. In dieser Halteposition H können die Knochenschrauben 2 auch trotz ihrer horizontalen Anordnung, welche in der Lagerungsposition L vorliegt, nicht aus dem Träger 1 herausfallen. Zur Aufrichtung des Aufnahmemoduls 53 wird dieses zunächst um die Drehachse D gedreht (vgl. Figuren 17a und 17b). Anschliessend wird das Aufnahmemodul in der Senkrichtung G abgesenkt, so dass die Eingriffsvorsprünge 61 in die Langlochabschnitte 59 eindringen. Somit wird die in den Figuren 19a bis 19c wiedergegebene Bereitstellungsposition B erreicht. Statt Aufnahmemodulen für Schrauben können selbstverständlich auch andere Halterungen wie z.B. eine Bohreraufnahme im Behälter 38 befestigt werden.

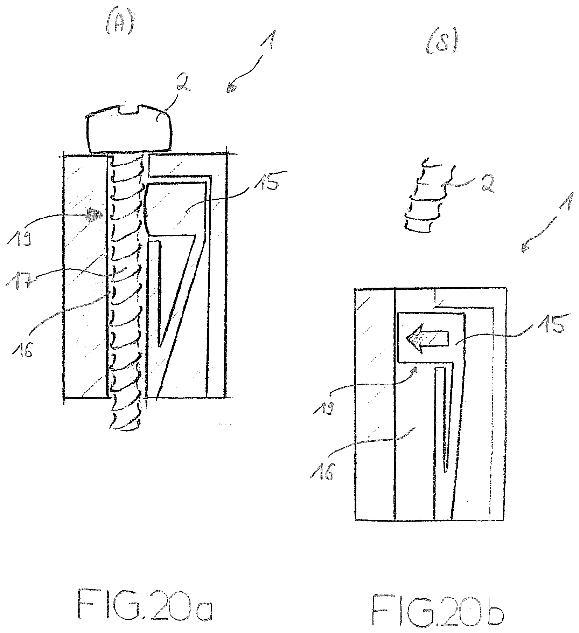

- In den Figuren 20a und 20b ist eine alternative Ausführungsform eines Trägers 1 mit Sperrmitteln 15 dargestellt, welche das Wiedereinsetzen einer einmal entnommenen Knochenschraube 2 verhindern. In der in Figur 13a dargestellten Aufnahmeposition A der Sperrmittel 15 ist die Knochenschraube 2 in den Träger 1 eingesetzt. In dieser Aufnahmeposition A befindet sich der Schaft 17 der Knochenschraube 2 in einer Aufnahmeöffnung 16 des Trägers 1. Die als Federelement 15 ausgebildeten Sperrmittel dringen in der Aufnahmeposition A nur so weit in einen Sperrbereich 19 in der Aufnahmeöffnung ein, dass der Schaft 17 in den Sperrbereich 19 der Aufnahmeöffnung 16 eindringt. Bei der Entnahme der Knochenschraube 2 dringt das Federelement 15 derart weit in den Sperrbereich 19 in der Aufnahmeöffnung 16 ein, dass der Schaft 17 der Knochenschraube 2 nicht mehr in den Sperrbereich 19 der Aufnahmeöffnung 16 eindringen kann. Auf diese Weise bewegt sich das Federelement 15 in die Sperrposition S, die in der Figur 13b dargestellt ist. In dieser Sperrposition S ist die Knochenschraube 2 nicht mehr in den Träger 1 einsetzbar. Somit wird das Wiedereinsetzen einer bereits entnommenen und möglicherweise beschädigten und/oder verschmutzten Knochenschraube 2 verhindert.

## Patentansprüche

1. Träger (1) zur Aufnahme von mindestens einer Knochenschraube (2) und/oder von mindestens einem Knochennagel, mit einer Halterung (3) für die Knochenschraube (2) bzw. den Knochennagel,
wobei
▪ die Halterung (3) in eine Halteposition (H) und in eine Freigabeposition (F) bringbar ist,
▪ die Knochenschraube (2) bzw. der Knochennagel in der Halteposition (H) in der Halterung (3) haltbar ist und
▪ die Halterung (3) bei der Ausübung einer Kraft und/oder eines Drehmoments auf die Knochenschraube (2) bzw. den Knochennagel und/oder auf die Halterung (3) in einer ersten Richtung (R1) von der Halteposition (H) in die Freigabeposition (F) bringbar ist,
**dadurch gekennzeichnet, dass**
▪ der Träger (1) ein Basiselement (5) aufweist, welches relativ zur Halterung (3) bewegbar ist,
▪ die Halterung (3) in der ersten Richtung (R1) relativ zum Basiselement (5) bewegbar ist und
▪ die Knochenschraube (2) bzw. der Knochennagel in der Freigabeposition (F) in einer der ersten Richtung (R1) im Wesentlichen entgegengesetzten zweiten Richtung (R2) entnehmbar ist.

2. Träger (1) gemäss Anspruch 1,
**dadurch gekennzeichnet, dass**
der Träger (1), insbesondere die Halterung (3), mindestens eine Haltefläche (4) aufweist, die in der Halteposition (H) in Kontakt mit der Knochenschraube (2) bzw. den Knochennagel ist oder insbesondere durch eine Bewegung der Knochenschraube (2) bzw. den Knochennagel derart in Kontakt mit der Knochenschraube (2) oder dem Knochennagel bringbar ist, dass die Knochenschraube (2) bzw. der Knochennagel im Träger (1) gehalten wird.

3. Träger (1) gemäss einem der Ansprüche 1 und 2,
**dadurch gekennzeichnet, dass**
die Halterung (3) bei der Ausübung einer Druckkraft auf die Knochenschraube (2) bzw. den Knochennagel in der ersten Richtung (R1) von der Halteposition (H) in die Freigabeposition (F) bringbar ist.

4. Träger (1) gemäss einem der Ansprüche 1 bis 3,
**gekennzeichnet durch**
Federmittel (6), mittels welcher die Halterung (3) zumindest in der Halteposition (H) am Basiselement (5) festklemmbar ist.

5. Träger (1) gemäss einem der Ansprüche 1 bis 4,
**dadurch gekennzeichnet, dass**
die Halterung (3) bei der Ausübung einer Kraft auf die Knochenschraube (2) bzw. den Knochennagel in der ersten Richtung (R1) derart relativ zum Basiselement (5) bewegbar ist, dass die Halterung (3) von der Halteposition (H) in die Freigabeposition (F) bringbar ist.

6. Träger (1) gemäss einem der Ansprüche 1 bis 5,
**gekennzeichnet durch**
Sollbruchmittel (79), welche bei der Bewegung von der Halteposition (H) in die Freigabeposition (F) zerstörbar sind.

7. Träger (1) gemäss einem der Ansprüche 1 bis 6, **dadurch gekennzeichnet, dass** die Halterung (3) eine Kontaktfläche (7) und das Basiselement (5) eine Gegenkontaktfläche (8) aufweist, wobei die Kontaktfläche (7) und die Gegenkontaktfläche (8) zumindest in der Halteposition (H) in Kontakt sind und die Kontaktfläche (7) und die Gegenkontaktfläche (8) derart aufeinander abgestimmt sind, dass die Halterung (3) bei der Ausübung einer Kraft auf die Knochenschraube (2) bzw. den Knochennagel in der ersten Richtung (R1) mittels der Federmittel (6) in die Freigabeposition (F) bewegbar sind.

8. Träger (1) gemäss Anspruch 7,
**dadurch gekennzeichnet, dass**
die Kontaktfläche (7) als Oberfläche mindestens eines Nockens (9) und/oder einer Abschrägung ausgebildet ist.

9. Träger (1) gemäss einem der Ansprüche 1 bis 8,
**dadurch gekennzeichnet,**
**dass** der Träger (1) Kennzeichnungen (14), insbesondere eine Herstellerangabe und/oder eine Produktnummer und/oder Seriennummer und/oder technische Spezifikationen, aufweist.

10. Chirurgischer Behälter (38) mit mindestens einem Träger (1) gemäss einem der Ansprüche 1 bis 9.

## Claims

1. A carrier (1) for receiving at least one bone screw (2) and/or at least one bone nail, with a holding device (3) for the bone screw (2) or the bone nail,
wherein
• the holding device (3) can be brought into a holding position (H) and into a release position (F),
• in the holding position (H), the bone screw (2) or the bone nail can be held in the holding device (3) and
• the holding device (3) can be brought from the holding position (H) into the release position (F) in the first direction (R1) when a force and/or torque is exerted on the bone screw (2) or the bone nail and/or on the holding device (3),
**characterized in that**
• the carrier (1) has a base element (5) which is movable relative to the holding device (3),
• the holding device (3) is movable in a first direction (R1) relative to the base element (5), and
• the bone screw (2) or bone nail is removable in the release position (F) in a second direction (R2) substantially opposite the first direction (R1).

2. The carrier (1) according to claim 1,
**characterized in that**
the carrier (1), in particular the holding device (3), has at least one holding surface (4) which, in the holding position (H), is in contact with the bone screw (2) or the bone nail or, in particular, can be brought into contact with the bone screw (2) or bone nail by a movement of the bone screw (2) or the bone nail such that the bone screw (2) or the bone nail is held in the carrier (1).

3. The carrier (1) according to one of claims 1 and 2,
**characterized in that**
the holding device (3) can be brought from the holding position (H) into the release position (F) when a compressive force is exerted on the bone screw (2) or the bone nail in the first direction (R1).

4. The carrier (1) according to one of claims 1 to 3,
**characterized by**
spring means (6) by means of which the holding device (3) can be clamped to the base element (5) at least in the holding position (H).

5. The carrier (1) according to one of claims 1 to 4,
**characterized in that**
the holding device (3), when a force is exerted on the bone screw (2) or the bone nail, is movable in the first direction (R1) relative to the base element (5) in such a way that the holding device (3) can be brought from the holding position (H) into the release position (F).

6. The carrier (1) according to one of claims 1 to 5,
**characterized by**
predetermined breaking means (79) which can be destroyed during the movement from the holding position (H) to the release position (F).

7. The carrier (1) according to one of claims 1 to 6,
**characterized in that**
the holding device (3) has a contact surface (7) and the base element (5) has a counter-contact surface (8), the contact surface (7) and the counter-contact surface (8) being in contact at least in the holding position (H) and the contact surface (7) and the counter-contact surface (8) being matched to one another in such a way that the holding device (3), when a force is exerted on the bone screw (2) or the bone nail, is movable in the first direction (R1) into the release position (F) by means of the spring means (6) .

8. The carrier (1) according to claim 7,
**characterized in that**
the contact surface (7) is formed as the surface of at least one cam (9) and/or a bevel.

9. The carrier (1) according to one of claims 1 to 8,
**characterized in that**
the carrier (1) contains markings (14), in particular a manufacturer's indication and/or a product number and/or serial number and/or technical specifications.

10. A surgical container (38) with at least one carrier (1) according to one of claims 1 to 9.

## Revendications

1. Porteur (1) pour recevoir au moins une vis à os (2) et/ou au moins un clou à os, avec un support (3) pour la vis à os (2) ou le clou à os,
où
• le support (3) peut être amené en position d'attente (H) et en position de libération (F),
• la vis à os (2) ou le clou à os peuvent être maintenus en position d'attente (H) dans le support (3) et
• le support (3) peut être amené de la position d'attente (H) à la position de libération (F) dans une première direction (R1) lorsqu'une force et/ou un couple est exercé sur la vis à os (2) ou le clou à os et/ou sur le support (3),
**caractérisé en ce que**
• le porteur (1) comporte un élément de base (5) qui est mobile par le support (3),
• le support (3) est amovible dans la première direction (R1) par rapport à l'élément de base (5) et
• la vis à os (2) ou le clou à os est amovible en position de libération (F) dans une deuxième direction (R2) sensiblement opposée à la première direction (R1).

2. Le porteur (1) selon la revendication 1,
**caractérisé en ce que**
le porteur (1), en particulier le support (3), présente au moins une surface de support (4) qui, en position d'attente (H), est en contact avec la vis à os (2) ou le clou à os ou, en particulier, peut être mise en contact avec la vis à os (2) ou du clou à os par un mouvement de la vis à os (2) ou du clou à os de telle sorte que la vis à os (2) ou le clou à os est supporté dans le porteur (1).

3. Le porteur (1) selon l'une des revendications 1 et 2,
**caractérisé en ce que**
le support (3) peut être amené de la position d'attente (H) à la position de libération (F) lorsqu'une force de compression est exercée sur la vis à os (2) ou le clou à os dans la première direction (R1).

4. Le porteur (1) selon l'une des revendications 1 à 3,
**caractérisé par**
des moyen de ressort (6) au moyen desquels le support (3) peut être serré sur l'élément de base (5) au moins dans la position d'attente (H).

5. Le porteur (1) selon l'une des revendications 1 à 4,
**caractérisé en ce que**
le support (3), lorsqu'une force est exercée sur la vis à os (2) ou le clou à os, est amovible dans la première direction (R1) par rapport à l'élément de base (5) de telle sorte que le support (3) peut être amené de la position d'attente (H) à la position de libération (F).

6. Le porteur (1) selon l'une des revendications 1 à 5,
**caractérisé par**
des moyens de rupture prédéterminés (79) qui peuvent être détruits lors du mouvement de la position d'attente (H) à la position de libération (F).

7. Le porteur (1) selon l'une des revendications 1 à 6,
**caractérisé en ce que**
le support (3) présente une surface de contact (7) et l'élément de base (5) une surface de contre-contact (8), la surface de contact (7) et la surface de contre-contact (8) étant en contact au moins dans la position d'attente (H) et la surface de contact (7) et la surface de contre-contact (8) étant adaptées l'une à l'autre de telle sorte que le support (3), lorsqu'une force est exercée sur la vis à os (2) ou le clou à os, est amovible dans la première direction (R1) ) en position de libération (F) à l'aide des moyen de ressort (6).

8. Le porteur (1) selon la revendication 7,
**caractérisé en ce que**
la surface de contact (7) est formée par la surface d'au moins un encoche (9) et/ou un biseau.

9. Le porteur (1) selon l'une des revendications 1 à 8,
**caractérisé en ce**
**que** le porteur (1) comporte des marquages (14), notamment une indication du fabricant et/ou un numéro de produit et/ou un numéro de série et/ou des spécifications techniques.

10. Conteneur chirurgical (38) avec au moins un porteur (1) selon l'une des revendications 1 à 9.
